(19) Europäisches Patentamt
European Patent Office
Office européen des brevets

(11) EP 4 442 262 A1

(12) **EUROPEAN PATENT APPLICATION**
published in accordance with Art. 153(4) EPC

(43) Date of publication:
**09.10.2024 Bulletin 2024/41**

(21) Application number: **22900454.4**

(22) Date of filing: **29.11.2022**

(51) International Patent Classification (IPC):
***A61K 31/7048*** (2006.01) ***A61K 31/34*** (2006.01)
***A61P 31/16*** (2006.01) ***A61P 31/14*** (2006.01)
***A61P 29/00*** (2006.01) ***A61P 11/00*** (2006.01)
***C07H 1/00*** (2006.01)

(52) Cooperative Patent Classification (CPC):
**A23L 33/105; A23L 33/125; A61K 31/34; A61K 31/7048; A61K 36/634; A61P 11/00; A61P 29/00; A61P 31/14; A61P 31/16; C07H 1/00; C07H 15/26;** Y02P 20/55

(86) International application number:
**PCT/CN2022/134969**

(87) International publication number:
**WO 2023/098650 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.12.2021 CN 202111447121**

(71) Applicant: **Ge, Xin**
**Dalian, Liaoning 116100 (CN)**

(72) Inventors:
- **WANG, Shuo**
  **Dalian, Liaoning 116100 (CN)**
- **LU, Mingming**
  **Dalian, Liaoning 116100 (CN)**
- **LIN, Rongxin**
  **Dalian, Liaoning 116100 (CN)**
- **YANG, Zifeng**
  **Dalian, Liaoning 116100 (CN)**
- **ZHONG, Nanshan**
  **Dalian, Liaoning 116100 (CN)**
- **SUI, Dayun**
  **Dalian, Liaoning 116100 (CN)**
- **YANG, Wei**
  **Dalian, Liaoning 116100 (CN)**
- **MA, Qinhai**
  **Dalian, Liaoning 116100 (CN)**
- **LU, Kesi**
  **Dalian, Liaoning 116100 (CN)**
- **GE, Xin**
  **Dalian, Liaoning 116100 (CN)**

(74) Representative: **Murgitroyd & Company**
**165-169 Scotland Street**
**Glasgow G5 8PL (GB)**

(54) **ANTIVIRAL USE OF BISEPOXYLIGNAN COMPOUND, AND BISEPOXYLIGNAN COMPOSITION AND PREPARATION OF BISEPOXYLIGNAN COMPOUND**

(57) The present invention provides an application of a bisepoxylignan compound or a bisepoxylignan composition in prevention and treatment of infections with influenza viruses, parainfluenza viruses, COVID-19 viruses and COVID-19 virus variants, an application thereof in prevention and treatment of pulmonary hemorrhage caused by the COVID-19 viruses and the COVID-19 virus variants (e.g., Delta), an application thereof in anti-inflammatory effects on pneumonia caused by influenza viruses, parainfluenza viruses, COVID-19 viruses and COVID-19 virus variants. In addition, the present invention further provides a chemical synthesis method of a bisepoxylignan compound lianqiaoxinside.

EP 4 442 262 A1

## Description

### Technical Field

The present invention belongs to the field of medical technology and medicinal chemistry. In particular, the present invention includes an preventive effect of a bisepoxylignan compound and a bisepoxylignan composition on infections with influenza viruses, parainfluenza viruses, COVID-19 viruses and many COVID-19 virus variants, a therapeutic effect thereof on influenza viruses, parainfluenza viruses, COVID-19 viruses and many COVID-19 virus variants, a therapeutic effect thereof on pulmonary hemorrhage caused by COVID-19 viruses and many COVID-19 virus variants, a therapeutic effect thereof on pneumonia caused by influenza viruses, parainfluenza viruses, COVID-19 viruses and many COVID-19 virus variants, a synthesis method of lianqiaoxinside used herein, etc.

### Background Art

Flu viruses are referred to as influenza viruses which are divided into three types: A, B, and C, and influenza viruses that have only been discovered in recent years will be classified as a type D. Typical clinical symptoms are acute hyperthermia, general aching, significant fatigue, and respiratory symptoms. Influenza viruses are mainly spread through airborne droplets, contact between susceptible people and infected people, or contact with contaminated items. Generally, autumn and winter are high-incidence seasons. Human parainfluenza viruses (HPIVs) often cause lower respiratory tract infections in children, recurrent upper respiratory tract infections (e.g., cold and sore throat), and recurrent lower respiratory tract diseases (e.g., pneumonia, bronchitis, and bronchiolitis), and often infect older people and immunocompromised people. The COVID-19 virus, a virus that causes coronavirus disease 2019 (also known as the novel coronavirus, SARS-CoV-2), belongs to coronaviruses. The COVID-19 virus continues to evolve into variants, including Alpha (B.1.1.7), Beta (B.1.351), Gamma (P.1), Delta (B.1.617.2) and other representative variants, and their variations further complicate clinical prevention and treatment (see Paulo R S Sanches, et al. Recent advances in SARS-CoV-2 Spike protein and RBD mutations comparison between new variants Alpha (B.1.1.7, United Kingdom), Beta (B.1.351, South Africa), Gamma (P.1, Brazil) and Delta ( B.1.617.2, India). J Virus Erad, 7(3):100054; Kaiming Tao, et al. The biological and clinical significance of emerging SARS-CoV-2 variants. Nat Rev Genet,17:1-17). In recent years, the epidemic of influenza viruses, parainfluenza viruses, COVID-19 viruses and their mutated viruses has become more and more serious on human health. At the same time, the occurrence of inflammations has also been bothering humans.

Fructus Forsythiae is dried fruits of Forsythia suspense (Thunb.) Vahl of Forsythia of Oleaceae, which are mainly distributed in Henan, Shanxi, Shaanxi, Shandong and other places in China, and also in Hubei, Hebei, Sichuan, and Gansu. It is often used for the treatment of acute wind-heat cold, carbuncle swelling and sores, lymph node tuberculosis, urinary tract infections and other symptoms. The main components of Fructus Forsythiae are bisepoxylignan components, such as phillyrin, lianqiaoxinside (molecular formula: $C_{26}H_{32}O_{11}$, chemical name:(7,8,7',8')-7,7'-bisepoxy-5'-hydroxy-3,3'hydroxy-bismethoxylignan, 4-O-β-D-glucoside), phillygenin (also known as ((+)-phillygenin), etc., which are present in the flowers, leaves, fruits, stem barks and root barks of Fructus Forsythiae, or may also be obtained by chemical synthesis, biotransformation, and extraction and purification processes. Their structural formulas are as follows:

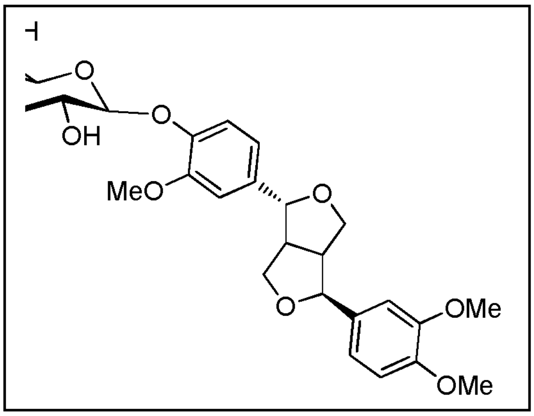

phillyrin

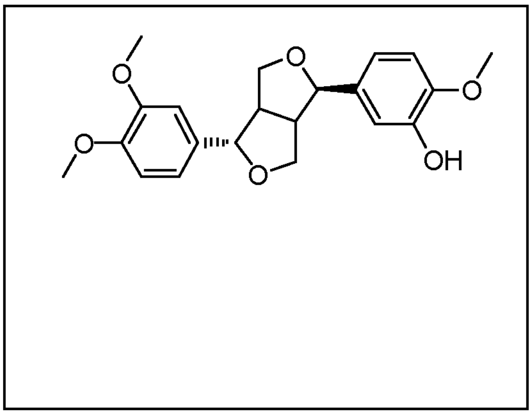

(+)-phillygenin

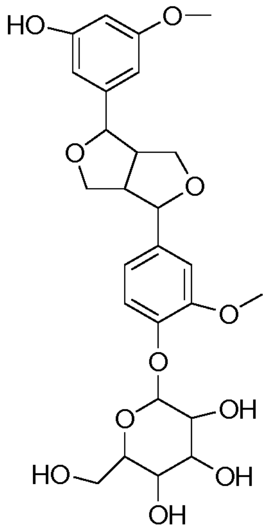

lianqiaoxinside.

In recent years, the inventors have developed phillygenin sulfate (see Chinese patent CN201310580801.5), phillygenin glucuronate (see Chinese patent CN201510320579.4) and phillygenin ibuprofenate (see Chinese patent CN201410387045.9) by means of chemical synthesis. Their structural formulas are as follows:

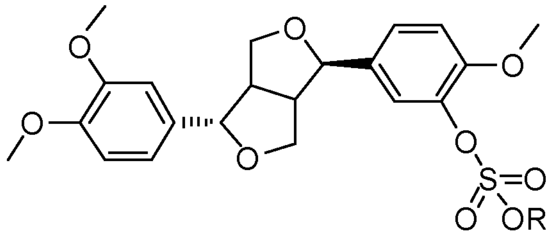

phillygenin sulfate

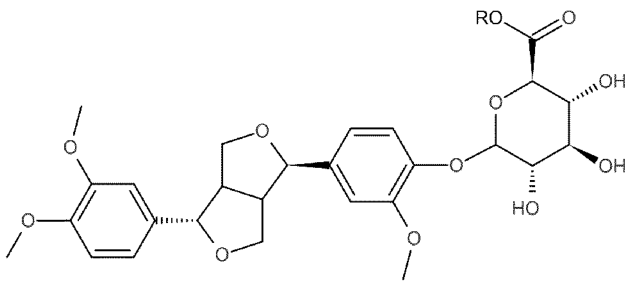

phillygenin glucuronate

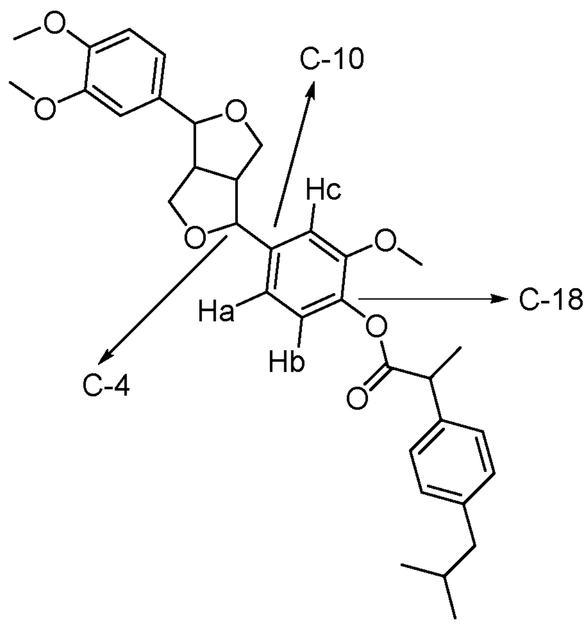

phillygenin ibuprofenate.

Phillyrin has antiviral, antibacterial and antioxidant properties, free radical scavenging and other pharmacological effects. Phillygenin has antioxidant, hypolipidemic, free radical scavenging, antibacterial, antitumor, antiviral and anti-inflammatory effects. Lianqiaoxinside has antioxidant and anti-inflammatory effects. Phillygenin sulfate, phillygenin glucuronate and phillygenin ibuprofenate have antiviral effects.

A bisepoxylignan compound lianqiaoxinside and bisepoxylignan compositions have not been involved in the published research literatures on the prevention and treatment of influenza and parainfluenza viruses, the bisepoxylignan compositions including: a component consisting of lianqiaoxinside and one of phillyrin, phillygenin, phillygenin sulfate, phillygenin glucuronate or phillygenin ibuprofenate respectively, a composition consisting of phillyrin and one of phillygenin sulfate, phillygenin glucuronate and phillygenin ibuprofenate respectively, and a composition formed by any two of phillygenin, phillygenin sulfate, phillygenin glucuronate and phillygenin ibuprofenate.

Bisepoxylignan compounds that have not been involved in the published research literatures on the prevention and treatment of COVID-19 viruses include: a single composition of and a compound formed by any two of lianqiaoxinside, phillygenin, phillygenin sulfate, phillygenin glucuronate and phillygenin ibuprofenate, or a composition consisting of phillyrin and lianqiaoxinside, or a composition consisting of phillyrin and phillygenin ibuprofenate. Bisepoxylignan compounds which have not been involved in the published research literatures on the prevention and treatment of COVID-19 mutated viruses include: phillyrin, lianqiaoxinside, phillygenin, phillygenin sulfate, phillygenin glucuronate, phillygenin ibuprofenate and other bisepoxylignan compositions, or a component consisting of phillyrin or phillygenin and one of lianqiaoxinside, phillygenin sulfate, phillygenin glucuronate and phillygenin ibuprofenate respectively, or a composition formed by any two of lianqiaoxinside, phillygenin sulfate, phillygenin glucuronate and phillygenin ibuprofenate.

The bisepoxylignan compounds or compositions discovered by the present inventors can inhibit influenza viruses, parainfluenza viruses, COVID-19 viruses and COVID-19 virus variants, and have obvious in-vitro virus inhibition effects; have obvious therapeutic effects on disease caused by viruses, and obviously reduced degree of lesion compared with positive control drug groups (a positive control drug for influenza is Oseltamivir phosphate, and an inflammatory control drug for COVID-19 and virus variants thereof is Remdesivir); have comparable therapeutic effect at a dose of 80 mg/kg to Remdesivir on Delta virus-infected mice, and have better pathological improvements for pulmonary hemorrhage and interstitial pneumonia than Remdesivir; can significantly inhibit lung indexes of mice infected with influenza and parainfluenza viruses in a 13.0 mg/kg dose group, and have an inhibition rate significantly higher than that of an Oseltamivir phosphate group; and can reduce the hemagglutination titers of mice infected with influenza and parainfluenza in a high-dose group and a low-dose group, and have a comparable effect at a low dose of 3.25 mg/kg to Oseltamivir phosphate. They have better pathological improvements than Oseltamivir phosphate in terms of pulmonary hemorrhage and interstitial pneumonia; have significant preventive effects on viral infections, and can prevent the adsorption and entry of influenza viruses and parainfluenza viruses into cells, with a blocking effect of more than 75%; have comparable preventive effects to Remdesivir against COVID-19 viruses and variants thereof produced at a dose of 80 mg/kg; and have an outstanding anti-inflammatory effect on the treatment of pneumonia caused by influenza viruses and COVID-19 viruses, a better anti-inflammatory effect at a dose of 26 mg/kg than Oseltamivir phosphate, and a better anti-COVID-19 pneumonia effect at a dose of 80mg/kg than Remdesivir. In summary, the bisepoxylignan compounds or compositions discovered by the present inventors have a prospect of becoming drugs for the prevention and treatment of diseases caused by influenza viruses, parainfluenza viruses, COVID-19 viruses and virus variants thereof.

In addition, a synthesis method of lianqiaoxinside has not been mentioned in the published literatures. The present inventors have designed a lianqiaoxinside synthesis route, which is convenient for the source of raw materials, cheap and easy to obtain, low in preparation cost, and capable of being industrially produced, thereby providing a new possibility for the source of drugs and broadening an application prospect of lianqiaoxinside drugs.

## Summary of the Invention

The technical problem to be solved by the present invention is to provide a new chemical synthesis method of lianqiaoxinside. Meanwhile, the technical problem to be solved by the present invention further includes: providing an preventive application of a bisepoxylignan compound or a bisepoxylignan composition on influenza viruses, parainfluenza viruses, COVID-19 viruses and many variants thereof, a therapeutic effect thereof on diseases caused by influenza viruses, parainfluenza viruses, COVID-19 viruses and many variants thereof, a therapeutic application thereof on pulmonary hemorrhage caused by COVID-19 viruses and many variants thereof, a therapeutic application thereof on pneumonia caused by influenza viruses, parainfluenza viruses, COVID-19 viruses and many variants thereof.

Specifically, in a first aspect, the present invention provides a synthesis method of lianqiaoxinside. The method includes the following steps:

step 1), obtaining corresponding β-keto esters (a compound of formula 4) and (a compound of formula 9) by taking vanillin (a compound of formula 1) and 3-hydroxy-5 methoxybenzaldehyde (a compound of formula 6) as raw materials via phenolic hydroxyl protection, oxidization and Claisen condensation reaction; then converting the β-keto esters into corresponding sodium salt (a compound of formula 5) and bromide (a compound of formula 10) respectively, and then performing a coupling reaction on the two fragments to obtain an intermediate (a compound of formula 11);

step 2), obtaining corresponding aglycone (a compound of formula 14) from the intermediate (the compound of formula 11) via a reduction reaction and a ring-closing reaction;

step 3) obtaining a target product from the aglycone (the compound of formula 14) via glycosylation, deacylation and silicyl removal; and

a chemical reaction formula is as follows:

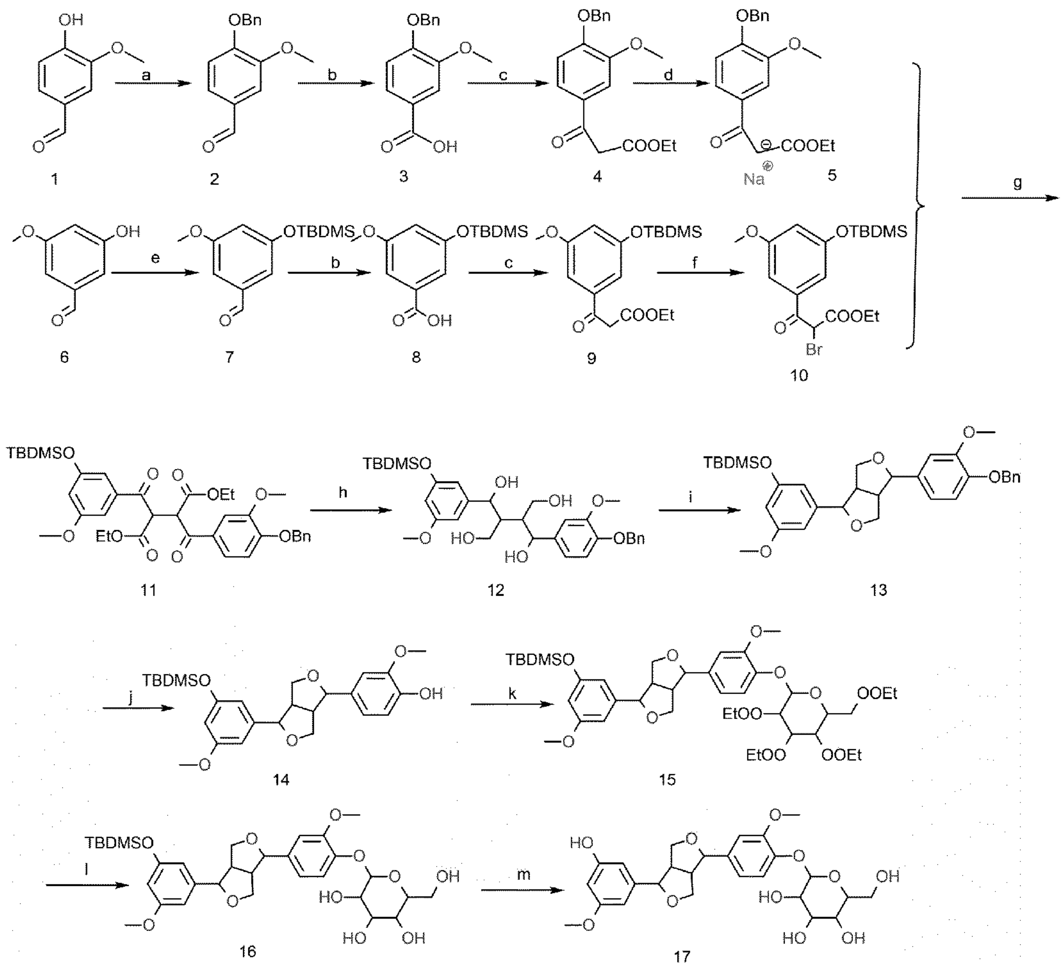


1 2 3 4 5 6 7 8 9 10 11 12 13 14 15 16 17

in which: a. benzyl chloride; b. potassium permanganate; c. thionyl chloride/ethyl acetoacetate/sodium ethoxide; d. sodium ethoxide; e. tert-butyldimethylchlorosilane; f. bromine water; g. methylene chloride; h. lithium aluminum hydride; i. methylsulfonyl chloride/pyridine; j. palladium carbon/hydrogen; k. acetylglucosyl trichloroethanimidate; 1. sodium methoxide; and m. tetrabutylammonium fluoride.

Preferably,
in step 1), synthesis of a compound of formula 2 includes: dissolving vanillin in acetonitrile, adding potassium carbonate, potassium iodide and benzyl chloride, and performing a reflux reaction for 0.5-24 h (preferably, 5 h) to obtain the compound of formula 2.

In step 1), a phenolic hydroxyl protecting group may be a silyl ether protecting group, an alkyl ether protecting group, an alkoxymethyl ether protecting group, or an ester protecting group, preferably an alkyl ether protecting group or benzyl chloride, and further preferably, benzyl chloride.

In step 1), a dissolution solvent of vanillin is acetonitrile, methanol or ethanol, preferably acetonitrile.

In step 1), synthesis of a compound of formula 3 includes: dissolving the compound of formula 2 in hot water, adding an oxidant potassium permanganate, and reacting at 70-80°C for 1 h to obtain the compound of formula 3.

The oxidant is potassium permanganate, hydrogen peroxide or chromium trioxide, preferably potassium permanganate; the reaction temperature is 40-100°C, preferably 60-90°C, and further preferably 70-80°C; and the reaction time is 0.2-5 h, preferably 0.5-2 h, and further preferably 1 h. In step 1), synthesis of a compound of formula 4 includes: dissolving the compound of formula 3 in thionyl chloride, reacting at 95°C for 8 h, removing thionyl chloride, adding anhydrous tetrahydrofuran and a sodium salt of ethyl acetoacetate, refluxing for 8 h, removing tetrahydrofuran, then adding ammonium chloride and 95% ethanol solution containing 1% ammonia water directly to residues, and refluxing for 4 h to obtain the compound of formula 4. Acyl chloride is oxalyl chloride or thionyl chloride, preferably thionyl chloride; the reflux temperature is 50-110°C, preferably 90-100°C, and further preferably 95°C; the reaction time is 4-12 h, preferably 6-10 h, and further preferably 8 h; when the solvent is tetrahydrofuran, the reaction time is 4-12 h, preferably 6-10 h, and further preferably 8 h; and when the solvent is a 95% ethanol solution, the reaction time is 2-6 h, preferably 3-5 h, and further preferably 4 h.

In step 1), synthesis of a compound of formula 7 includes: dissolving 3-hydroxy-5 methoxybenzaldehyde in N,N-dimethylformamide, adding imidazole and tert-butyldimethylchlorosilane, and reacting at 35°C for 10 h to obtain the compound of formula 7. The phenolic hydroxyl protecting group includes a silyl ether protecting group, an alkyl ether protecting group, an alkoxymethyl ether protecting group or an ester protecting group, preferably the silyl ether protecting group, and further preferably tert-butyldimethylchlorosilane; the solvent is selected as N,N-dimethylformamide, N,N-dimethylacetamide, tetrahydrofuran, or dichloromethane, preferably N,N-dimethylformamide; the reaction temperature is 10-50°C, preferably 20-40°C, and further preferably 35°C; and the reaction time is 8-12 h, preferably 10 h. In step 1), synthesis of a compound of formula 8 includes: dissolving the compound of formula 7 in hot water, adding potassium permanganate, and reacting at 70-80°C for 1 h to obtain the compound of formula 8.

The oxidant is potassium permanganate, hydrogen peroxide, chromium trioxide and the like, preferably potassium permanganate; the reaction temperature is 50-100°C, preferably 60-90°C, and further preferably 70-80°C; and the reaction time is 0.2-3 h, preferably 0.5-2 h, and further preferably 1 h.

In step 1), synthesis of a compound of formula 9 includes: dissolving the compound of formula 8 in thionyl chloride, reacting at 95°C for 8 h, removing thionyl chloride by distillation, adding anhydrous tetrahydrofuran and a sodium salt of ethyl acetoacetate, refluxing for 8 h, removing tetrahydrofuran, then adding ammonium chloride and 95% ethanol solution containing 1% ammonia water directly to residues, and refluxing for 4 h to obtain the compound of formula 9. Acyl chloride is oxalyl chloride or thionyl chloride, preferably thionyl chloride; when the solvent is thionyl chloride, the reflux temperature is 80-110°C, preferably 90-100°C, and further preferably 95°C; the reaction time is 4-12 h, preferably 6-10 h, and further preferably 8 h; when the solvent is tetrahydrofuran, the reaction time is 4-12 h, preferably 6-10 h, and further preferably 8 h; and when the solvent is a 95% ethanol solution, the reaction time is 2-6 h, preferably 3-5 h, and further preferably 4 h.

In step 1), synthesis of a compound of formula 11 includes: adding the compound of formula 4 to an absolute ethanol solution dissolved with the same amount of metal sodium, and stirring and reacting at room temperature for 3 h to obtain the compound of formula 5; dissolving the compound of formula 9 in an anhydrous tetrahydrofuran solution, adding bromine water, and stirring and reacting at room temperature for 3 h to obtain a compound of formula 10; and dissolving the compound of formula 5 and the compound of formula 10 in a dichloromethane solution, and stirring and reacting at room temperature for 10 h to obtain the compound of formula 11.

A strong base is an anhydrous ethanol solution of sodium metal, an anhydrous methanol solution of sodium metal or a sodium hydroxide solution, preferably the anhydrous ethanol solution of sodium metal; the halogen is bromine water or iodine, preferably bromine water; the reaction time of the compound of formula 4 is 1-6 h, preferably 2-5 h, and further preferably 3 h; and the reaction time of the compound of formula 9 is 5-15 h, preferably 8-12 h, and further preferably 10 h; and

the reaction time of the compound of formula 5 and the compound of formula 10 is 5-15 h, preferably 8-12 h, and further preferably 10 h.

In step 2), synthesis of a compound of formula 12 includes: dissolving the compound of formula 11 in anhydrous tetrahydrofuran, adding an anhydrous tetrahydrofuran solution containing lithium aluminum hydride, performing a reflux reaction for 6 h, and stirring at room temperature overnight to obtain the compound of formula 12.

A reducing agent is lithium aluminum hydride or bis(2-methoxyethoxy) aluminum hydride, preferably lithium aluminum hydride; and the reflux reaction time is 3-9 h, preferably 4-8 h, and further preferably 6 h.

In step 2), synthesis of a compound of formula 13 includes: dissolving the compound of formula 12 in re-distilled benzene, adding pyridinium p-toluenesulfonate salt powder, and performing a reflux reaction for 3 h to obtain the compound of formula 13.

A dehydrating agent is selected as pyridine tosylate or concentrated sulfuric acid, and preferably pyridine tosylate; the solvent is toluene or benzene, preferably benzene; and the reaction time is 1-6 h, preferably 2-4 h, and further preferably 3 h.

In step 2), synthesis of a compound of formula 14 includes: dissolving the compound of formula 13 in methanol under a hydrogen condition, adding 10% palladium carbon, and stirring and reacting at room temperature for 10 h to obtain the compound of formula 14.

A reducing agent is selected as 5% palladium carbon, 10% palladium carbon or 20% palladium carbon, and preferably 10% palladium carbon; and the solvent is methanol or ethanol, n-butanol or isopropanol, preferably methanol; the temperature is 10-40°C, preferably 20-30°C, and further preferably 25°C; and the reaction time is 5-15 h, preferably 8-12 h, and further preferably 10 h.

In step 3), synthesis of a compound of formula 15 includes: dissolving the compound of formula 14 in anhydrous dichloromethane under the protection of nitrogen, adding 2,3,4,6-tetra-O-acyl-D-glucopyranosyl trichloroethanimidate, trimethylsilyl trifluoromethanesulfonate and a 4Å-type aluminosilicate molecular sieve, and stirring and reacting at 0°C for 10 h to obtain the compound of formula 15.

An inert shielding gas is nitrogen, argon or helium, preferably nitrogen; a glycosyl donor 2,3,4,6-tetra-O-acyl-D-glucopyranosyl trichloroethanimidate is selected as 2,3,4,6-tetra-O-acetyl-D-glucopyranosyl trichloroethanimidate or

2,3,4,6-tetra-O-benzoyl-D-glucopyranosyl trichloroethanimidate; a Lewis acid catalyst is selected as one or more of $C_3$-$C_9$ haloamide, $C_2$-$C_8$silicyl fluoroalkyl sulfonate, $C_1$-$C_6$ silver fluoroalkyl sulfonate or a boron trifluoride-ether complex, preferably N-iodosuccinimide, silver trifluoromethanesulfonate, trimethylsilicone trifluoromethanesulfonate or boron trifluoride-ether complex, and further preferably silver trifluoromethanesulfonate, trimethylsilicone trifluoromethanesulfonate or boron trifluoride-ether complex; a molecular sieve is selected as a 3Å-5Å type aluminosilicate molecular sieve, preferably a 4Å type aluminosilicate molecular sieve; the solvent is selected as dichloromethane, chloroform, 1,2-dichloroethane or toluene, preferably dichloromethane; the temperature is 0-20°C, preferably 0-10°C, and further preferably 0°C; and the reaction time is 4-15 h, preferably 8-10 h, and further preferably 10 h.

In step 3), synthesis of a compound of formula 16 includes: dissolving the compound of formula 15 in a mixed solution of dichloromethane and methanol, adding sodium methoxide, and stirring and reacting at 25°C for 4 h to obtain the compound of formula 16.

A ratio of a volume of dichloromethane to a volume of methanol in the mixed solution of solvent dichloromethane and methanol is 1: (1-10), preferably 1: 2; and the reaction time is 4-12 h, preferably 4 h.

In step 3), a synthesis condition of the compound of formula 17 includes: dissolving the compound of formula 16 in anhydrous tetrahydrofuran, adding tetrabutylammonium fluoride, and stirring and reacting at 35°C for 3 h to obtain the compound of formula 17.

The solvent is dichloromethane or tetrahydrofuran, preferably tetrahydrofuran; the reaction temperature is 20-40°C, preferably 35°C; and the reaction time is 1-5 h, preferably 3 h.

In a specific embodiment of the present invention, the application in the first aspect of the present invention is to prepare lianqiaoxinside by a synthesis method, with a yield of 90%, and a content of 99.95%.

In a second aspect, the present invention provides an application of a bisepoxylignan compound or a bisepoxylignan composition in the preparation of health food or drug for prevention or treatment of infections with influenza viruses, parainfluenza viruses, COVID-19 viruses or COVID-19 virus variants.

The present invention further provides an application of a bisepoxylignan compound or a bisepoxylignan composition in the preparation of health food or drug for prevention or treatment of pulmonary hemorrhage caused by COVID-19 viruses or COVID-19 virus variants.

In addition, the present invention further provides an application of a bisepoxylignan compound or a bisepoxylignan composition in the preparation of health food or drug for resisting pneumonia caused by influenza viruses, parainfluenza viruses, COVID-19 viruses and COVID-19 virus variants.

Antiviral and therapeutic effects may be in-vivo and in-vitro virus inhibition effects, viral inflammation resistance effects, improved lung damages caused by viral inflammations and protective effects on lung tissues.

The COVID-19 virus variant has a mutation on the basis of original COVID-19 virus. Preferably, in the application of the second aspect of the present invention, the COVID-19 virus variant is a COVID-19 virus variant Beta or a COVID-19 virus variant Delta.

Unless otherwise indicated herein, the bisepoxylignan compound is basically pure, e.g., 95-99.9% pure.

Unless otherwise indicated herein, the orders of the components in the bisepoxylignan composition may be used interchangeably, i.e., the composition is measured as a whole. Preferably, in the present invention, a weight ratio of each component in the composition is 0.1-99.9%; preferably, a weight ratio of one component in the composition is 80-99.9%, and a weight ratio of another one or more components is 0.1-20%; and more preferably, a weight ratio of one component in the composition is 90-99.9%, and a weight ratio of another one or more components is 0.1-10%.

In a specific embodiment of the present invention, the application in the second aspect of the present invention is an application in the preparation of a drug for the treatment of diseases caused by influenza viruses, parainfluenza viruses and COVID-19 virus variants. That is, the bisepoxylignan compound and composition are administrated after the occurrence or knowledge of infections with influenza viruses, parainfluenza viruses and COVID-19 virus variants.

The bisepoxylignan composition may be used in combination with other drugs against influenza viruses, parainfluenza viruses, COVID-19 viruses and virus variants thereof, or drugs for the treatment of diseases caused by influenza viruses, parainfluenza viruses, COVID-19 viruses and virus variants thereof, or used alone. Preferably, the application in the second aspect of the present invention is an application of the bisepoxylignan compound or the bisepoxylignan composition as a sole active ingredient. For example, the sole active ingredient in the drug is the bisepoxylignan compound or composition. That is, the second aspect of the present invention preferably provides an application of a bisepoxylignan compound or composition as a sole drug active ingredient in the preparation of drugs against influenza viruses, parainfluenza viruses, COVID-19 viruses and virus variants thereof, or an application of the bisepoxylignan compound or composition as a sole drug active ingredient in the preparation of drugs for the prevention or treatment of influenza viruses, parainfluenza viruses, COVID-19 viruses and virus variants thereof.

As described herein, the drug may be formulated into a pharmaceutical preparation including a pharmaceutically acceptable carrier. This is well known to those skilled in the art. Preferably, in the application of the present invention, the drug is present in the form of tablets, capsules, pills, powders, granules, a syrup, a solution, an emulsion, an injection, a spray, an aerosol, a gel, a cream, cataplasm, a rubber plaster or a plaster. In the drug, the content of the bisepoxylignan

compound is 0.1-99.9%. A ratio of a total weight of the bisepoxylignan compound or the bisepoxylignan composition to a weight of the pharmaceutically acceptable carrier is 1:1 to 1:100. The preparation method of the drug generally includes a step of mixing the bisepoxylignan compound or the bisepoxylignan composition and the pharmaceutically acceptable carrier.

As described herein, the health food may be prepared into a health food product by including a carrier acceptable to food science. This is well known to those skilled in the art. Preferably, in the application of the present invention, the health food is present in the food form of tablets, capsules, pills, powders, granules, a syrup, a solution, an emulsion, a spray, an aerosol, a gel, a cream, cataplasm, a rubber plaster or a plaster, candies and preserved fruits, tea, a beverage, baked food or a wine product. In the health food, the content of the bisepoxylignan is 0.1-99.9%. A ratio of a total weight of the bisepoxylignan compound or the bisepoxylignan composition to a weight of the carrier acceptable to food science is 1:1 to 1:100. The preparation method of the health food generally includes a step of mixing the bisepoxylignan compound or the bisepoxylignan composition and the carrier acceptable to food science.

The bisepoxylignan compound is a bisepoxylignan monomer compound. For example, the bisepoxylignan compound may be phillyrin, lianqiaoxinside, phillygenin, phillygenin sulfate, phillygenin glucuronate, or phillygenin ibuprofenate. The bisepoxylignan compound is prepared by means of chemical synthesis, biotransformation or extraction and purification processes. In a specific embodiment, lianqiaoxinside is prepared by means of the synthesis method in the first aspect of the prevent invention.

The bisepoxylignan composition is a composition consisting of phillyrin and one or more of lianqiaoxinside, phillygenin sulfate, phillygenin glucuronate and phillygenin ibuprofenate respectively. For example, the bisepoxylignan composition is a composition consisting of lianqiaoxinside and phillyrin, a composition consisting of phillyrin and phillygenin sulfate, a composition consisting of phillyrin and phillygenin glucuronate, a composition consisting of phillyrin and phillygenin ibuprofenate, a composition consisting of phillyrin, lianqiaoxinside and phillygenin sulfate, or a composition consisting of lianqiaoxinside, phillyrin and phillygenin ibuprofenate.

The bisepoxylignan composition is a composition consisting of any two or more of lianqiaoxinside, phillygenin, phillygenin sulfate, phillygenin glucuronate and phillygenin ibuprofenate. For example, the bisepoxylignan composition is a composition consisting of lianqiaoxinside and phillygenin, a composition consisting of lianqiaoxinside and phillygenin sulfate, a composition consisting of lianqiaoxinside and phillygenin glucuronate, a composition consisting of lianqiaoxinside and phillygenin ibuprofenate, a composition consisting of phillygenin and phillygenin sulfate, a composition consisting of phillygenin and phillygenin glucuronate, a composition consisting of phillygenin and phillygenin ibuprofenate, a composition of phillygenin sulfate and phillygenin glucuronate, a composition consisting of phillygenin sulfate and phillygenin ibuprofenate, a composition consisting of phillygenin glucuronate and phillygenin ibuprofenate, or a composition consisting of phillygenin, lianqiaoxinside and phillygenin glucuronate.

The bisepoxylignan composition is a mixture prepared by means of chemical synthesis, biotransformation or extraction and purification processes, or a mixture prepared by matching monomer compounds prepared by means of chemical synthesis, biotransformation or extraction and purification processes. The proportion of each component in the composition is 0.1-99.9%.

In another specific embodiment of the present invention, the present invention provides a preventive effect of a bisepoxylignan compound and composition on influenza viruses, parainfluenza viruses, COVID-19 viruses or many variants thereof. That is, the bisepoxylignan compound or composition is administrated before the occurrence or knowledge of infections with influenza viruses, parainfluenza viruses and original COVID-19 virus or variants thereof.

In a specific embodiment of the present invention, the application of the present invention is an application in the preparation of drugs for the prevention of diseases caused by influenza viruses, parainfluenza viruses, original COVID-19 virus and variants thereof. Preferably, the bisepoxylignan compound or composition is a sole active ingredient in the drug.

In another specific embodiment of the present invention, the present invention provides a therapeutic application of a bisepoxylignan compound or composition on pulmonary hemorrhage caused by COVID-19 viruses or many variants thereof. That is, the bisepoxylignan compound or composition is administrated after the occurrence or knowledge of infections with original COVID-19 virus or variants thereof.

In a specific embodiment of the present invention, the application of the present invention is an application in the preparation of drugs for the treatment of pulmonary hemorrhage caused by original COVID-19 virus and variants thereof. Preferably, the bisepoxylignan compound or composition is a sole active ingredient in the drug.

In another specific embodiment of the present invention, the present invention provides an anti-inflammatory effect of a bisepoxylignan compound and composition, that is, an application on pneumonia caused by influenza viruses and COVID-19 Delta virus variants.

In a specific embodiment of the present invention, the application of the present invention is an inhibitory effect on pneumonia caused by influenza viruses and COVID-19 Delta virus variants. Preferably, the bisepoxylignan compound or composition is a sole active ingredient in the drug.

Correspondingly, in response to the second aspect of the present invention, the present invention further

provides second medicinal use of the bisepoxylignan compound and composition, and a prevention or treatment method using the bisepoxylignan compound and composition. For example, the present invention provides a bisepoxylignan compound and composition, which are used for resisting influenza viruses, parainfluenza viruses, COVID-19 viruses or variants thereof. The present invention further provides a bisepoxylignan compound and composition, which are used for the prevention or treatment of diseases caused by influenza viruses, parainfluenza viruses, COVID-19 viruses and variants thereof. For example, the present invention provides a method for resisting influenza viruses, parainfluenza viruses, COVID-19 viruses or variants thereof, including administrating an effective amount of bisepoxylignan compound or composition to an individual (e.g., experimental animal or human) in need. Correspondingly, the present invention further provides a method for the prevention or treatment of diseases caused by influenza viruses, parainfluenza viruses, COVID-19 viruses and variants thereof, including administrating an effective amount of bisepoxylignan compound or composition to an individual (e.g., experimental animal or human) in need.

As described herein, a dose (effective amount) and form of administration are generally determined by a physician based on patient's specific circumstances (e.g., age, weight, gender, duration of illness, physical condition, and severity of infection). As the specific situation of the patient changes, the dose of administration will also vary, so the selected amount is within a range of physician's abilities. The form of administration is determined according to a dosage form of a drug composition, and suitable forms of administration are oral, parenteral, mucosal, intramuscular, intramuscular, intravenous, subcutaneous, intraocular or intradermal or transdermal forms of administration, preferably the oral administration form.

The present invention has the beneficial effects: a method for resisting influenza viruses, parainfluenza viruses, COVID-19 viruses and many variants thereof is provided, which contributes to the better prevention and control of increasingly complex epidemic virus epidemics; the bisepoxylignan compound and composition can effectively prevent and treat diseases caused by influenza viruses, parainfluenza viruses, COVID-19 viruses and many variants thereof, which can effectively treat pulmonary hemorrhage caused by COVID-19 viruses, effectively inhibit the development of inflammations and provide new prevention and treatment methods for uninfected and infected people; and a new synthesis route of lianqiaoxinside is provided, which is cheap and available in raw materials, low in preparation cost, and capable of being industrially produced. For ease of understanding, the present invention will be further described in detail below in conjunction with specific examples. It should be particularly noted that these descriptions are only exemplary and do not constitute any limitation on the scope of the present invention. According to the discussion of the present Description, many changes and alterations of the present invention are obvious to those skilled in the art. In addition, the present invention has cited published literatures, which are intended to describe the present invention more clearly and incorporated herein for reference by their entities, as if their entities have been repeated herein.

**Detailed Description of the Invention**

The present invention is further illustrated by the examples below. Unless otherwise specified, the methods used in the examples are recorded in the technical literatures in the art and in the specification documents of the drug regulatory agencies, and the instruments, raw materials and reagents are purchased in the open market.

**Example 1 Synthesis and preparation method of lianqiaoxinside**

1. Vanillin (15.2 g, 100 mmol) was dissolved in 100 ml of acetonitrile, added with potassium carbonate (6.9 g, 50 mmol), potassium iodide (0.66 g, 4 mmol) and benzyl chloride (14 g, 110 mmol), subjected to a reflux reaction for 5 h, quenched with saturated ammonium chloride, reduced in pressure to remove acetonitrile, extracted with dichloromethane, spin-dried and allowed to pass through a column to obtain a compound of formula 2, with a yield of 92%;
2. the compound of formula 2 (21.8 g, 92 mmol) was dissolved in 200 ml of hot water, added with potassium permanganate (24.1 g, 150 mmol), reacted at 70-80°C for 1 h, and alkalized to pH = 10; filtrate was acidified and subjected to suction filtration to obtain a compound of formula 3, with a yield of 90%;
3. the compound of formula 3 (20.6 g, 81 mmol) was added with 50 ml of thionyl chloride, reacted at 95°C for 8 h, distilled to remove thionyl chloride, and added with 50 ml of anhydrous tetrahydrofuran; this solution was dropwise added to the same amount of 100 ml of tetrahydrofuran solution of sodium salt of ethyl acetoacetate, and refluxed for 8 h; tetrahydrofuran was removed; then ammonium chloride (8 g) and 100 ml of 95% ethanol solution containing 1% ammonia were directly added to residues, refluxed for 4 h, reduced in pressure to remove ethanol, extracted with dichloromethane, spin-dried and allowed to pass through a column to obtain a compound of formula 4, with a yield of 75%;
4. 3-hydroxy-5-methoxybenzaldehyde (15.2 g, 100 mmol) was dissolved in 30 ml of N,N-dimethylformamide, added with imidazole (13.6 g, 200 mmol) and tert-butyldimethylchlorosilane (16.6 g, 110 mmol), reacted at 35°C for 10 h, then quenched with saturated ammonium chloride, extracted with dichloromethane, spin-dried and allowed to pass through a column to obtain a compound of formula 7, with a yield of 94%;

5. the compound of formula 7 (24.1 g, 92 mol) was dissolved in hot water, added with potassium permanganate (24.1 g, 150 mmol), reacted at 70-80°C for 1 h, and alkalized to pH = 10; filtrate was acidified and subjected to suction filtration to obtain a compound of formula 8, with a yield of 93%;
6. the compound of formula 8 (22.6 g, 83 mmol) was added with 50 ml of thionyl chloride, reacted at 95°C for 8 h, distilled to remove thionyl chloride, and added with 50 ml of anhydrous tetrahydrofuran; this solution was dropwise added to the same amount of 100 ml of tetrahydrofuran solution of sodium salt of ethyl acetoacetate, and refluxed for 8 h; tetrahydrofuran was removed; then ammonium chloride (8 g) and of 100 ml 95% ethanol solution containing 1% ammonia were directly added to residues, refluxed for 4 h, reduced in pressure to remove ethanol, extracted with dichloromethane, spin-dried and allowed to pass through a column to obtain a compound of formula 9, with a yield of 72%;
7. the compound of formula 4 (17.1 g, 52 mmol) was added to 150 ml of absolute ethanol solution dissolved with metal sodium (1.2 g, 52 mmol), stirred and reacted at room temperature for 3 h, and distilled at reduced pressure to remove ethanol to obtain a compound of formula 5; the compound of formula 9 (17.6 g, 50 mmol) was added in an anhydrous tetrahydrofuran solution, added with bromine water (11.5 g, 72 mmol), stirred and reacted at room temperature for 3 h and distilled at reduced pressure to remove tetrahydrofuran to obtain a compound of formula 10; and the compound of formula 5 and the compound of formula 10 were dissolved in 100 ml of dichloromethane solution, stirred and reacted at room temperature for 3 h, spin-dried and allowed to pass through a column to obtain a compound of formula 11, with a yield of 65%;
8. the compound of formula 11 (20.3 g, 31 mmol) was dissolved in 100 ml of anhydrous tetrahydrofuran, added with an anhydrous tetrahydrofuran solution containing 15 g of lithium aluminum hydride, subjected to a reflux reaction for 6 h, stirred at room temperature overnight, added with 100 ml of ethyl acetate to decompose excess lithium aluminum hydride, adjusted with concentrated hydrochloric acid to neutral, spin-dried and allowed to pass through a column to a compound of formula 12, with a yield of 82%;
9. the compound of formula 12 (11.3 g, 21 mmol) was dissolved in 20 mL of re-distilled benzene, added with pyridinium p-toluenesulfonate salt powder, subjected to a reflux reaction for 3 h, then cooled to room temperature, directly spin-dried and allowed to pass through a column to obtain a compound of formula 13, with a yield of 32%;
10. the compound of formula 13 (3.0 g, 6 mmol) was dissolved in 20 mL of methanol under a hydrogen condition, added with 2 g of 10% palladium carbon, stirred and reacted at room temperature for 3 h, filtered, spin-dried and allowed to pass through a column to obtain a compound of formula 14, with a yield of 90%;
11. the compound of formula 14 (2.1 g, 5 mmol) was dissolved in anhydrous dichloromethane under the protection of nitrogen, added with 2,3,4,6-tetra-O-acyl-D-glucopyranosyl trichloroethanimidate (3.7 g, 7.5 mmol), trimethylsilyl trifluoromethanesulfonate (0.3 ml, 15 mmol) and a 4Å-type aluminosilicate molecular sieve (4.2 g), stirred and reacted at 0°C for 10 h, quenched with triethylamine (1.5 mmol), filtered, spin-dried and allowed to pass through a column to obtain a compound of formula 15, with a yield of 72%;
12. the compound of formula 15 (2.2 g, 3 mmol) was dissolved in a mixed solution of dichloromethane and methanol (a volume ratio of 1: 2), added with sodium methoxide, stirred and reacted at 25°C for 4 h, adjusted with acetic acid to pH=7, spin-dried and allowed to pass through a column to obtain a compound of formula 16, with a yield of 90%; and
13. the compound of formula 16 (1.1 g, 2 mmol) was dissolved in anhydrous tetrahydrofuran, added with tetrabutylammonium fluoride, stirred and reacted at 35°C for 3 h, quenched with saturated ammonium chloride, extracted with dichloromethane, spin-dried and allowed to pass through a column to obtain a compound of formula 17, with a yield of 90%. The purity in a high-performance liquid chromatography test was 99.95%.
14. An m/z 543.18309 peak in a high-resolution mass spectrum of the compound of the present invention was a [M+Na]+ peak of a sample, so the compound had a molecular weight of 520, and the NMR data was as follows:
Complete assignment of chemical structure $^{13}$CNMR spectrum:

| Serial No. | $^{13}$C chemical shift (δ) | 1H-related chemical shift (δ) | Structural signs |
|---|---|---|---|
| 1 | 148.90 | / | 16 |
| 2 | 147.26 | / | 2 |
| 3 | 145.87 | / | 17 |
| 4 | 145.25 | / | 6 |
| 5 | 135.28 | / | 14 |
| 6 | 129.58 | / | 4 |
| 7 | 118.11 | 6.86 | 18 |
| 8 | 117.87 | 6.74 | 3 |
| 9 | 115.14 | 7.04, 6.74 | 1,19 |
| 10 | 110.38 | 6.96 | 15 |
| 11 | 109.73 | 6.89 | 5 |

(continued)

| Serial No. | $^{13}C$ chemical shift (δ) | 1H-related chemical shift (δ) | Structural signs |
|---|---|---|---|
| 12 | 100.10 | 4.88 | 21 |
| 13 | 86.64 | 4.37 | 11 |
| 14 | 81.36 | 4.76 | 8 |
| 15 | 77.00 | 3.27 | 25 |
| 16 | 76.84 | 3.27 | 22 |
| 17 | 73.19 | 3.27 | 23 |
| 18 | 70.23 | 4.08, 3.74 | 10 |
| 19 | 69.65 | 3.16 | 24 |
| 20 | 68.95 | 3.74,3.11 | 13 |
| 21 | 60.66 | 3.67,3.45 | 26 |
| 22 | 55.64 | 3.77 | 20 |
| 23 | 55.56 | 3.76 | 7 |
| 24 | 53.99 | 2.82 | 12 |
| 25 | 49.33 | 3.35 | 9 |

$^1$HNMR data: $^1$HNMR(600MHz,d6-DMSO)δ: 8.85(br,1H,-OH), 7.04 (d,1H,J=8.49Hz,-CH=), 6.96(d,1H,J=1.94Hz,-CH=), 6.89(s,1H,-CH=), 6.86(dd,1H,J=8.49Hz,1.94Hz,-CH=), 6.74(d,2H.J=0.77Hz,-CH=), 5.19 (d,1H,J=4.92Hz,-OH), 5.05(d,1H,J=4.47Hz,-OH), 4.99(d,1H,J=5.36Hz,-OH), 4.88(d,1H,J=7.45Hz,>OH-), 4.76(d,1H,J=5.81Hz,>OH-), 4.51(t,1H,J=5.51Hz,
-OH), 4.37(d,1H,J=6.85Hz,>OH-), 4.08(d,1H,J=9.39Hz, -CH2-), 3.77
(s,3H,-OCH3), 3.76(s,3H,-OCH3), 3.74(m,2H,-CH2-), 3.67(m,1H, -CH2-), 3.45(m,1H,-CH2-), 3.35(m,1H ,>OH-), 3.27(m,3H,>OH-), 3.16(m,1H,>OH-), 3.11(t,1H,J=8.49Hz,-CH2-), 2.82(m,1H,>OH-).

Therefore, it can be determined that the compound synthesized in this experiment is the illustrated structure:

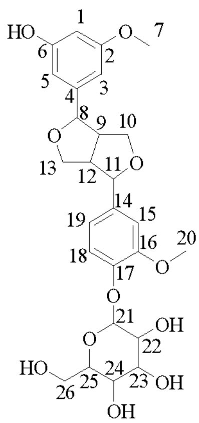

**Example 2 Experimental study of in-vitro efficacy of drugs of the present invention against COVID-19 virus and variants thereof**

**1 In-vitro antiviral test**

**1.1 Test materials**

(1) Drugs

Bisepoxylignan compounds include: lianqiaoxinside (having a purity of 99.95%), phillyrin (having a purity of 99.96%), phillygenin (having a purity of 99.96%), phillygenin sulfate (having a purity of 99.95%), phillygenin glucuronate (having a purity of 99.95%), or phillygenin ibuprofenate (having a purity of 99.95%).

Bisepoxylignan compositions include: lianqiaoxinside/phillyrin, lianqiaoxinside/phillygenin, lianqiaoxinside/phillygenin sulfate, lianqiaoxinside/phillygenin glucuronate, lianqiaoxinside/phillygenin ibuprofenate, phillyrin/phillygenin sulfate, phillyrin/phillygenin glucuronate, phillyrin/phillygenin ibuprofenate, phillygenin/phillygenin sulfate, phillygenin/phillygenin glucuronate, phillygenin/phillygenin ibuprofenate, phillygenin sulfate/phillygenin glucuronate, phillygenin sulfate/phillygenin ibuprofenate, phillygenin glucuronate/phillygenin ibuprofenate, phillyrin/lianqiaoxinside/phillygenin sulfate, lianqiaoxinside/phillyrin/phillygenin ibuprofenate, and phillygeninllianqiaoxinside/phillygenin glucuronate.

The above test drugs were provided by Dalian Fusheng Natural Medicine Development Co., Ltd.

Positive drug: (1) Remdesivir, provided by Guangzhou Institute of Biomedicine and Health, Chinese Academy of Sciences (synthesized by Professor Zhang Jiancun's team), specification: 1 g. Preparation method: during an in-vitro test, DMSO was prepared into mother liquor which was aliquoted, stored at 4°C for later use and diluted to a required concentration with a cell working solution before use. During an in-vivo test, 0.5% sodium carboxymethyl cellulose was prepared into a required concentration. (2) Oseltamivir phosphate, China Institute for the Control of Pharmaceutical and Biological Products. Product batch number: 101096-201901, 100 mg/piece as a positive control drug in this test.

(2) Cell line: Vero E6 cells (African green monkey kidney cells), State Key Laboratory of Respiratory Diseases, Guangzhou Institute of Respiratory Medicine.

(3) Virus strains: COVID-19 virus strain, COVID-19 virus Beta variant, and COVID-19 virus Delta variant: P3 Laboratory of Guangzhou Customs Technology Center (Laboratory of Highly Pathogenic Microorganisms of the State Key Laboratory of Respiratory Diseases).

1.2 Experimental method

The test was conducted in the BSL-3 Laboratory. A cell control group, a blank control (solvent control) group, a virus control (negative control) group, and tested drug groups of different concentrations were set. A VeroE6 cell suspension with a cell density of $2\times10^5$cells/mL was plated in a sterile 96-well culture plate respectively (100 μL/well), and cultured at 37°C, 5% $CO_2$ for 24 h; a culture supernatant was discarded, and a 100 TCID50 virus solution was added to an experimental group and a virus control group (100 μL/well), and then subjected to routine culture adsorption for 2 h; and a culture supernatant was discarded, 100 μL of drugs of different concentrations (250, 125, 62.5, 31.25, 15.62 μg/mL) were added to per well (100 μL/well, 3 replicates per concentration), and incubated for 3-4 days in routine culture. Cytopathogenic effect (CPE) was observed under a light microscope, and the degrees of cell lesions were recorded according to the following 6-level criteria: "-" means that the cells are free of lesions; "±" means that the degree of cell lesions was less than 10%; "+" means that the degree of cell lesions was about 25%; "++" means that the degree of cell lesions was about 50%; "+++" means that the degree of cell lesions was about 75%; and "++++" means that the degree of cell lesions was more than 75%. A median inhibitory concentration (IC50) and a selection index (SI) were calculated.

1.3 Experimental results

The results of Table 2-1 showed that the tested drugs had obvious inhibitory effects on both COVID-19 and variants thereof.

**Table 2-1 Results of in-vitro efficacy experiments of bisepoxylignan compounds and compositions against COVID-19 and variants thereof**

| Serial No. | Test drugs | Composition proportion | COVID-19 virus | | COVID-19 Beta virus | | COVID-19 Delta virus | |
|---|---|---|---|---|---|---|---|---|
| | | | $IC_{50}$ | SI | $IC_{50}$ | SI | $IC_{50}$ | SI |
| 1 | Lianqiaoxinside. | -- | 62.1 | 31.61 | 337.4 | 7.64 | 238.8 | 7.14 |
| 2 | Phillyrin | -- | 63.3 | 30.50 | 334.6 | 7.10 | 286.9 | 6.86 |
| 3 | Phillygenin | -- | 166.5 | 5.33 | 84.5 | 2.12 | 240.1 | 2.11 |
| 4 | Phillygenin sulfate | -- | 202.2 | 5.86 | 394.6 | 2.37 | 458.3 | 1.63 |
| 5 | Phillygenin glucuronate | -- | 164.5 | 2.95 | 274.6 | 2.96 | 336.3 | 1.16 |
| 6 | Phillygenin ibuprofenate. | -- | 161.8 | 4.72 | 276.8 | 3.43 | 339.0 | 1.79 |
| 7 | Lianqiaoxinside and phillyrin | 99.9 : 0.1 | 61.8 | 32.58 | 338.2 | 7.59 | 238.2 | 7.84 |
| 8 | Lianqiaoxinside and phillyrin | 0.1 : 99.9 | 62.9 | 32.47 | 335.0 | 7.99 | 286.2 | 6.79 |
| 9 | Lianqiaoxinside and phillygenin | 99.9 : 0.1 | 62.0 | 31.58 | 338.1 | 7.09 | 238.3 | 7.09 |

(continued)

| Serial No. | Test drugs | Composition proportion | COVID-19 virus | | COVID-19 Beta virus | | COVID-19 Delta virus | |
|---|---|---|---|---|---|---|---|---|
| | | | $IC_{50}$ | SI | $IC_{50}$ | SI | $IC_{50}$ | SI |
| 10 | Lianqiaoxinside and phillygenin | 0.1 : 99.9 | 166.3 | 5.96 | 85.1 | 2.10 | 240.3 | 2.10 |
| 11 | Lianqiaoxinside and phillygenin sulfate | 99.9 : 0.1 | 62.5 | 31.66 | 337.8 | 7.93 | 238.9 | 7.54 |
| 12 | Lianqiaoxinside and phillygenin sulfate | 0.1 : 99.9 | 201.8 | 5.35 | 394.9 | 2.94 | 458.9 | 1.60 |
| 13 | Lianqiaoxinside and phillygenin glucuronate | 99.9 : 0.1 | 66.4 | 31.54 | 89.1 | 7.58 | 243.0 | 7.81 |
| 14 | Lianqiaoxinside and phillygenin glucuronate | 0.1 : 99.9 | 164.3 | 2.75 | 275.0 | 2.90 | 336.1 | 1.10 |
| 15 | Lianqiaoxinside and phillygenin ibuprofenate | 99.9 : 0.1 | 62.5 | 32.64 | 337.7 | 7.67 | 238.6 | 8.01 |
| 16 | Lianqiaoxinside and phillygenin ibuprofenate | 0.1 : 99.9 | 162.3 | 4.92 | 277.0 | 3.40 | 338.8 | 1.93 |
| 17 | Phillyrin and phillygenin sulfate | 99.9 : 0.1 | 63.2 | 30.22 | 334.2 | 6.89 | 286.3 | 6.19 |
| 18 | Phillyrin and phillygenin sulfate | 0.1 : 99.9 | 202.6 | 5.97 | 394.5 | 2.30 | 458.8 | 1.71 |
| 19 | Phillyrin and phillygenin glucuronate | 99.9 : 0.1 | 62.8 | 30.73 | 334.3 | 7.09 | 286.6 | 6.73 |
| 20 | Phillyrin and phillygenin glucuronate | 0.1 : 99.9 | 163.9 | 2.87 | 274.3 | 2.97 | 336.6 | 1.20 |
| 21 | Phillyrin and phillygenin ibuprofenate | 99.9 : 0.1 | 63.6 | 30.42 | 334.5 | 7.88 | 286.7 | 6.58 |
| 22 | Phillyrin and phillygenin ibuprofenate | 0.1 : 99.9 | 162.3 | 4.82 | 276.6 | 3.57 | 338.6 | 1.64 |
| 23 | Phillygenin and phillygenin sulfate | 99.9 : 0.1 | 165.8 | 5.32 | 84.3 | 2.13 | 240.6 | 2.10 |
| 24 | Phillygenin and phillygenin sulfate | 0.1 : 99.9 | 202.6 | 5.92 | 395.0 | 2.88 | 458.7 | 1.60 |
| 25 | Phillygenin and phillygenin glucuronate | 99.9 : 0.1 | 165.8 | 5.37 | 85.2 | 2.42 | 240.4 | 2.74 |
| 26 | Phillygenin and phillygenin glucuronate | 0.1 : 99.9 | 164.4 | 3.03 | 274.9 | 2.90 | 336.2 | 1.10 |
| 27 | Phillygenin and phillygenin ibuprofenate | 99.9 : 0.1 | 165.7 | 6.05 | 84.6 | 2.54 | 240.7 | 2.56 |
| 28 | Phillygenin and phillygenin ibuprofenate | 0.1 : 99.9 | 161.8 | 5.14 | 276.7 | 3.40 | 338.6 | 1.92 |
| 29 | Phillygenin sulfate and phillygenin ibuprofenate | 99.9 : 0.1 | 202.0 | 6.01 | 395.0 | 2.64 | 458.5 | 1.53 |
| 30 | Phillygenin sulfate and phillygenin ibuprofenate | 0.1 : 99.9 | 162.1 | 4.95 | 277.0 | 3.53 | 338.4 | 1.70 |
| 31 | Phillygenin sulfate and phillygenin glucuronate | 99.9 : 0.1 | 202.6 | 5.85 | 395.1 | 2.31 | 458.6 | 1.62 |
| 32 | Phillygenin sulfate and phillygenin glucuronate | 0.1 : 99.9 | 164.4 | 3.41 | 274.4 | 2.85 | 336.6 | 1.54 |
| 33 | Phillygenin glucuronate and phillygenin ibuprofenate | 99.9 : 0.1 | 164.6 | 2.95 | 274.7 | 2.90 | 336.8 | 1.13 |

(continued)

| Serial No. | Test drugs | Composition proportion | COVID-19 virus | | COVID-19 Beta virus | | COVID-19 Delta virus | |
|---|---|---|---|---|---|---|---|---|
| | | | $IC_{50}$ | SI | $IC_{50}$ | SI | $IC_{50}$ | SI |
| 34 | Phillygenin glucuronate and phillygenin ibuprofenate | 0.1 : 99.9 | 161.9 | 5.53 | 276.2 | 3.66 | 338.9 | 1.58 |
| 35 | Phillyrin/lianqiaoxinside/ phillygenin | 99.8: 0.1: 0.1 | 63.0 | 34.23 | 335.0 | 7.47 | 286.4 | 7.01 |
| 36 | Lianqiaoxinside/phillyrin/ phillygenin | 99.8: 0.1: 0.1 | 62.6 | 35.42 | 337.1 | 7.69 | 238.7 | 6.97 |
| 37 | Phillygenin/ lianqiaoxinside/phillyrin | 99.8: 0.1: 0.1 | 165.8 | 5.93 | 84.9 | 2.53 | 240.2 | 2.64 |
| 38 | Phillyrin/lianqiaoxinside/ phillygenin sulfate | 99.8: 0.1: 0.1 | 62.9 | 32.57 | 334.5 | 7.87 | 286.4 | 7.15 |
| 39 | Lianqiaoxinside/phillyrin/ phillygenin ibuprofenate | 99.8: 0.1: 0.1 | 62.4 | 36.53 | 337.5 | 7.92 | 238.9 | 7.16 |
| 40 | Phillygenin/ lianqiaoxinside/ phillygenin glucuronate | 99.8: 0.1: 0.1 | 166.6 | 6.58 | 84.7 | 2.88 | 240.5 | 2.84 |

**Example 3 Efficacy test results of drugs of the present invention against influenza viruses and parainfluenza viruses in vitro**

**1 In-vitro antiviral test**

**1.1 Test materials**

(1) Drugs

Bisepoxylignan compounds include: lianqiaoxinside (having a purity of 99.95%), phillyrin (having a purity of 99.96%), phillygenin (having a purity of 99.96%), phillygenin sulfate (having a purity of 99.95%), phillygenin glucuronate (having a purity of 99.95%), or phillygenin ibuprofenate (having a purity of 99.95%).

Bisepoxylignan compositions include: lianqiaoxinside/phillyrin, lianqiaoxinside/phillygenin, lianqiaoxinside/phillygenin sulfate, lianqiaoxinside/phillygenin glucuronate, lianqiaoxinside/phillygenin ibuprofenate, phillyrin/phillygenin sulfate, phillyrin/phillygenin glucuronate, phillyrin/phillygenin ibuprofenate, phillygenin/phillygenin sulfate, phillygenin/phillygenin glucuronate, phillygenin/phillygenin ibuprofenate, phillygenin sulfate/phillygenin glucuronate, phillygenin sulfate/phillygenin ibuprofenate, phillygenin glucuronate/phillygenin ibuprofenate, phillyrin/lianqiaoxinside/phillygenin sulfate, lianqiaoxinside/phillyrin/phillygenin ibuprofenate, and phillygeninllianqiaoxinside/phillygenin glucuronate.

The above test drugs were provided by Dalian Fusheng Natural Medicine Development Co., Ltd. Oseltamivir phosphate: China Institute for the Control of Pharmaceutical and Biological Products. Product batch number: 101096-201901, 100 mg/piece as a positive control drug in this test.

The above drugs were dissolved in purified water, filtered, sterilized and aliquoted, and set aside at 4°C, which were the drugs to be tested in this test.

(2) Cell line: Vero (African green monkey kidney cells): School of Basic Medical Sciences, Jilin University.

(3) Virus strain: (1) influenza virus strains, parainfluenza virus strains: Institute of Virology, Chinese Academy of Preventive Medicine.

(4) Main equipment and reagents:

Biological Safety Cabinet: BHC-1300II.AB3, AIRTECH; CO2 Incubator: MCO-18AIC, SANYO; Inverted Microscope: CKX41, OLYMPUS; Electronic Analytical Balance: AR1140/C, DHAUS; Medium: DMEM, HyClone; Fetal Bovine Serum: HyClone; Trypsin: Gibco; MTT: Sigma; DMSO: Tianjin Beilian Fine Chemicals Development Co., Ltd.

1.2 Test method

(1) Cell preparation

Vero cells were subcultured for 1-2 d to make them into flakes, and trypsinized in the presence of clear boundaries, strong three-dimensional sense and strong refractive power, until needle-like holes appeared on the cell surfaces; after the trypsinization solution was absorbed completely, a few milliliters of culture medium were taken to blow away the cells; the cells were counted, diluted to about $5\times10^7$/L with a culture medium (DMEM containing 10% fetal bovine serum), and then inoculated in a 96-well culture plate; and the cells grew into a monolayer .

(2) Drug toxicity assay

Cytotoxicity test: the drugs were diluted at the concentrations shown in Table 3-1 for cytotoxicity assay.

**Table 3-1 Drug dilution reference table (unit: g/L)**

| Concentration gradients / Drugs | Gradient 1 | Gradient 2 | Gradient 3 | Gradient 4 | Gradient 6 | Gradient 7 | Gradient 8 |
|---|---|---|---|---|---|---|---|
| Test drugs | 1 | 0.5 | 0.25 | 0.125 | 0.03125 | 0.015625 | 0.078125 |
| Oseltamivir phosphate | 2 | 1 | 0.5 | 0.25 | 0.0625 | 0.03125 | 0.015625 |

Note: the test drugs were a bisepoxylignan compound and a bisepoxylignan composition.

The above drugs of different concentrations diluted with a maintenance solution (DMEM containing 2% fetal bovine serum) were added dropwise to Vero monolayer cells (0.2 ml/well, 6 replicate wells per concentration), and a 6-well normal control (a normal control group free of drug) and a 6-well blank control (a culture medium) were additionally provided and cultured in a 37°C, 5% CO2 incubator, and CPE was observed and recorded under an inverted microscope every day. After 72 h, 20 μL of MTT solution (5 mg·mL-1) was added to each well and continued to incubate for 4 h; the culture medium of each well was pipetted and discarded; 100 μL of DMSO was added to each well and shaken for 5 min; and an OD value was measured at 492 nm and the cell viability was calculated. In SPSS 18.0 statistical software, the cell viability was subjected to Probit regression analysis, and a maximum non-toxic concentration (TC0) and a median toxic concentration (TC50) of the drug to the Vero cells were calculated.

(3) Determination of influenza virus TCID50

The virus was diluted in 10-fold decrease to different dilutions of 10-1, 10-2, 10-3, 10-4, 10-5, and 10-6, and sequentially inoculated on a monolayer Vero cell 96-well culture plate (100 μL/well and 6 wells per dilution), and a normal cell control group was set at the same time. The virus was incubated at 37°C, 5% CO2 for 2 h, a virus solution was discarded, and 100 μL of cell maintenance solution was added per well, and cultured at 37 °C in 5% CO2. Cytopathic results were observed under the microscope at the beginning of Day 3, and results were determined and recorded on Days 7-8; and the virus titer was calculated using a Karber method by taking the maximum dilution that could cause positive lesions in 50% of cell wells as an endpoint.

Formula $\mathrm{LogTCID_{50}}=\mathrm{XM}+\frac{1}{2}\mathrm{d}-\mathrm{d}\frac{\Sigma Pi}{100}$ ($TCID_{50}$: 50% histiocyte infection dose; XM: logarithm of the maximum concentration dilution of the virus; d: logarithm of dilution factor (fold); Σpi: a sum of the percentages of lesions per dilution).

(4) Effect of drug on the cytopathogenic effects of virus

The culture plate that has been overgrown with monolayer cells was taken, and a culture solution was pipetted and discarded; the cells were inoculated at a virus attack amount corresponding to 100TCID50, adsorbed in a 37°C, 5% CO2 incubator for 2 h, added with a specific concentration (approximately the maximum non-toxic concentration) of each drug solution, and cultured in 6 duplicate wells per concentration (200 μL/well). Oseltamivir phosphate was provided as a positive drug control group, and meanwhile a normal control group (free of virus and drug) and a virus control group (a control group with virus but no drug) were provided; and the effect of the drug on virus-induced CPE was observed. After 72 h, an OD value was measured at a wavelength of 492 nm by a MTT colorimetric method, and the antiviral effective rate (ER%) of the drug was calculated. In SPSS 18.0 statistical software, an ANOVA method was used to

compare the significant differences in antiviral effective rates of various drugs.

ER% = (mean OD value of drug treatment group - mean OD value of virus control group) / (mean OD value of cell control group - mean OD value of virus control group) × 100%

### 1.3. Test results

**(1)**$TCID_{50}$

Influenza virus:

$$LogTCID_{50}=-2+0.5-\frac{100+100+50}{100}=-4$$

Parainfluenza virus:

$$LogTCID_{50}=-2+0.5-\frac{100+100+50}{100}=-4$$

**(2) Drug toxicity determination**

1) Determination of the cytotoxicity of drugs

A maximum non-toxic concentration ($TC_0$), a median toxic concentration ($TC_{50}$) and a concentration of each drug in antiviral experiments to Vero cells were shown in the following table.

**Table 3-2 Results of drug cytotoxicity experiment (unit: g/L)**

| **Serial No.** | **Test drugs** | **Composition Proportion** | **Maximum non-toxic concentration** | **Median toxic concentration** | **Experimental concentration** |
|---|---|---|---|---|---|
| | Oseltamivir phosphate | -- | 0.29 | 0.831 | 0.30 |
| 1 | Lianqiaoxinside. | -- | 0.0067 | 0.56 | 0.01 |
| 2 | Phillyrin | -- | 0.0067 | 0.56 | 0.01 |
| 3 | Phillygenin | -- | 0.012 | 0.31 | 0.02 |
| 4 | Phillygenin sulfate | -- | 0.012 | 0.31 | 0.01 |
| 5 | Phillygenin glucuronate | -- | 0.012 | 0.31 | 0.01 |
| 6 | Phillygenin ibuprofenate. | -- | 0.012 | 0.31 | 0.01 |
| 7 | Lianqiaoxinside and phillyrin | 99.9 : 0.1 | 0.0067 | 0.56 | 0.01 |
| 8 | Lianqiaoxinside and phillyrin | 0.1 : 99.9 | 0.0067 | 0.56 | 0.01 |
| 9 | Lianqiaoxinside and phillygenin | 99.9 : 0.1 | 0.0067 | 0.56 | 0.01 |
| 10 | Lianqiaoxinside and phillygenin | 0.1 : 99.9 | 0.012 | 0.31 | 0.02 |
| 11 | Lianqiaoxinside and phillygenin sulfate | 99.9 : 0.1 | 0.0067 | 0.56 | 0.01 |
| 12 | Lianqiaoxinside and phillygenin sulfate | 0.1 : 99.9 | 0.012 | 0.31 | 0.01 |
| 13 | Lianqiaoxinside and phillygenin glucuronate | 99.9 : 0.1 | 0.0067 | 0.56 | 0.01 |

(continued)

| Serial No. | Test drugs | Composition Proportion | Maximum non-toxic concentration | Median toxic concentration | Experimental concentration |
|---|---|---|---|---|---|
| 14 | Lianqiaoxinside and phillygenin glucuronate | 0.1 : 99.9 | 0.012 | 0.31 | 0.01 |
| 15 | Lianqiaoxinside and phillygenin ibuprofenate | 99.9 : 0.1 | 0.0067 | 0.56 | 0.01 |
| 16 | Lianqiaoxinside and phillygenin ibuprofenate | 0.1 : 99.9 | 0.012 | 0.31 | 0.01 |
| 17 | Phillyrin and phillygenin sulfate | 99.9 : 0.1 | 0.0067 | 0.56 | 0.01 |
| 18 | Phillyrin and phillygenin sulfate | 0.1 : 99.9 | 0.012 | 0.31 | 0.01 |
| 19 | Phillyrin and phillygenin glucuronate | 99.9 : 0.1 | 0.0067 | 0.56 | 0.01 |
| 20 | Phillyrin and phillygenin glucuronate | 0.1 : 99.9 | 0.012 | 0.31 | 0.01 |
| 21 | Phillyrin and phillygenin ibuprofenate | 99.9 : 0.1 | 0.0067 | 0.56 | 0.01 |
| 22 | Phillyrin and phillygenin ibuprofenate | 0.1 : 99.9 | 0.012 | 0.31 | 0.01 |
| 23 | Phillygenin and phillygenin sulfate | 99.9 : 0.1 | 0.012 | 0.31 | 0.02 |
| 24 | Phillygenin and phillygenin sulfate | 0.1 : 99.9 | 0.012 | 0.31 | 0.01 |
| 25 | Phillygenin and phillygenin glucuronate | 99.9 : 0.1 | 0.012 | 0.31 | 0.02 |
| 26 | Phillygenin and phillygenin glucuronate | 0.1 : 99.9 | 0.012 | 0.31 | 0.01 |
| 27 | Phillygenin and phillygenin ibuprofenate | 99.9 : 0.1 | 0.012 | 0.31 | 0.02 |
| 28 | Phillygenin and phillygenin ibuprofenate | 0.1 : 99.9 | 0.012 | 0.31 | 0.01 |
| 29 | Phillygenin sulfate and phillygenin ibuprofenate | 99.9 : 0.1 | 0.012 | 0.31 | 0.01 |
| 30 | Phillygenin sulfate and phillygenin ibuprofenate | 0.1 : 99.9 | 0.012 | 0.31 | 0.01 |
| 31 | Phillygenin sulfate and phillygenin glucuronate | 99.9 : 0.1 | 0.012 | 0.31 | 0.01 |
| 32 | Phillygenin sulfate and phillygenin glucuronate | 0.1 : 99.9 | 0.012 | 0.31 | 0.01 |
| 33 | Phillygenin glucuronate and phillygenin ibuprofenate | 99.9 : 0.1 | 0.012 | 0.31 | 0.01 |
| 34 | Phillygenin glucuronate and phillygenin ibuprofenate | 0.1 : 99.9 | 0.012 | 0.31 | 0.01 |
| 35 | Phillyrin/lianqiaoxinside/ phillygenin | 99.8: 0.1: 0.1 | 0.0067 | 0.56 | 0.01 |
| 36 | Lianqiaoxinside/phillyrin/ phillygenin | 99.8: 0.1: 0.1 | 0.0067 | 0.56 | 0.01 |

(continued)

| Serial No. | Test drugs | Composition Proportion | Maximum non-toxic concentration | Median toxic concentration | Experimental concentration |
|---|---|---|---|---|---|
| 37 | Phillygenin/ lianqiaoxinside/phillyrin | 99.8: 0.1: 0.1 | 0.012 | 0.31 | 0.02 |
| 38 | Phillyrin/lianqiaoxinside/ phillygenin sulfate | 99.8: 0.1: 0.1 | 0.0067 | 0.56 | 0.01 |
| 39 | Lianqiaoxinside/phillyrin/ phillygenin ibuprofenate | 99.8: 0.1: 0.1 | 0.0067 | 0.56 | 0.01 |
| 40 | Phillygenin/ lianqiaoxinside/phillygenin glucuronate | 99.8: 0.1: 0.1 | 0.012 | 0.31 | 0.02 |

2) The results of protective effects of drugs on virus-induced cytopathy

The bisepoxylignan compound and composition both had obvious inhibitory effects on influenza virus and parainfluenza virus, and had a higher effective rate than Oseltamivir phosphate. Specific data was shown in the following table.

**Table 3-3 Efficacy results of drugs against influenza and parainfluenza viruses (effective rate, ER%)**

| Serial No. | Test drugs | Composition Proportion | Influenza Virus | Parainfluenza Virus |
|---|---|---|---|---|
| | Oseltamivir phosphate | -- | 81.92 | 91.16 |
| 1 | Lianqiaoxinside. | -- | 89.02 | 99.92 |
| 2 | Phillyrin | -- | 85.94 | 94.43 |
| 3 | Phillygenin | -- | 83.10 | 91.41 |
| 4 | Phillygenin sulfate | -- | 92.38 | 99.92 |
| 5 | Phillygenin glucuronate | -- | 84.47 | 93.52 |
| 6 | Phillygenin ibuprofenate. | -- | 84.51 | 93.96 |
| 7 | Lianqiaoxinside and phillyrin | 99.9 : 0.1 | 88.77 | 97.35 |
| 8 | Lianqiaoxinside and phillyrin | 0.1 : 99.9 | 85.87 | 94.26 |
| 9 | Lianqiaoxinside and phillygenin | 99.9 : 0.1 | 88.51 | 97.86 |
| 10 | Lianqiaoxinside and phillygenin | 0.1 : 99.9 | 82.14 | 89.45 |
| 11 | Lianqiaoxinside and phillygenin sulfate | 99.9 : 0.1 | 88.42 | 98.66 |
| 12 | Lianqiaoxinside and phillygenin sulfate | 0.1 : 99.9 | 92.36 | 99.90 |
| 13 | Lianqiaoxinside and phillygenin glucuronate | 99.9 : 0.1 | 88.42 | 96.66 |
| 14 | Lianqiaoxinside and phillygenin glucuronate | 0.1 : 99.9 | 84.11 | 91.82 |
| 15 | Lianqiaoxinside and phillygenin ibuprofenate | 99.9 : 0.1 | 89.06 | 97.97 |
| 16 | Lianqiaoxinside and phillygenin ibuprofenate | 0.1 : 99.9 | 83.98 | 91.78 |
| 17 | Phillyrin and phillygenin sulfate | 99.9 : 0.1 | 85.56 | 93.62 |
| 18 | Phillyrin and phillygenin sulfate | 0.1 : 99.9 | 92.66 | 100.53 |
| 19 | Phillyrin and phillygenin glucuronate | 99.9 : 0.1 | 85.35 | 93.19 |
| 20 | Phillyrin and phillygenin glucuronate | 0.1 : 99.9 | 84.59 | 93.75 |
| 21 | Phillyrin and phillygenin ibuprofenate | 99.9 : 0.1 | 85.70 | 94.87 |
| 22 | Phillyrin and phillygenin ibuprofenate | 0.1 : 99.9 | 83.84 | 91.52 |
| 23 | Phillygenin and phillygenin sulfate | 99.9 : 0.1 | 82.40 | 90.94 |
| 24 | Phillygenin and phillygenin sulfate | 0.1 : 99.9 | 93.03 | 100.33 |
| 25 | Phillygenin and phillygenin glucuronate | 99.9 : 0.1 | 82.32 | 90.85 |
| 26 | Phillygenin and phillygenin glucuronate | 0.1 : 99.9 | 84.12 | 91.83 |
| 27 | Phillygenin and phillygenin ibuprofenate | 99.9 : 0.1 | 82.36 | 90.90 |
| 28 | Phillygenin and phillygenin ibuprofenate | 0.1 : 99.9 | 84.51 | 92.96 |

(continued)

| Serial No. | Test drugs | Composition Proportion | Influenza Virus | Parainfluenza Virus |
|---|---|---|---|---|
| 29 | Phillygenin sulfate and phillygenin ibuprofenate | 99.9 : 0.1 | 92.93 | 100.12 |
| 30 | Phillygenin sulfate and phillygenin ibuprofenate | 0.1 : 99.9 | 84.09 | 92.00 |
| 31 | Phillygenin sulfate and phillygenin glucuronate | 99.9 : 0.1 | 92.72 | 100.69 |
| 32 | Phillygenin sulfate and phillygenin glucuronate | 0.1 : 99.9 | 84.69 | 92.96 |
| 33 | Phillygenin glucuronate and phillygenin ibuprofenate | 99.9 : 0.1 | 83.91 | 91.40 |
| 34 | Phillygenin glucuronate and phillygenin ibuprofenate | 0.1 : 99.9 | 84.03 | 92.93 |
| 35 | Phillyrin/lianqiaoxinside/phillygenin | 99.8: 0.1: 0.1 | 85.15 | 92.77 |
| 36 | Lianqiaoxinside/phillyrin/phillygenin | 99.8: 0.1: 0.1 | 88.32 | 96.45 |
| 37 | Phillygenin/lianqiaoxinside/phillyrin | 99.8: 0.1: 0.1 | 82.38 | 90.92 |
| 38 | Phillyrin/lianqiaoxinside/phillygenin sulfate | 99.8: 0.1: 0.1 | 85.68 | 93.85 |
| 39 | Lianqiaoxinside/phillyrin/phillygenin ibuprofenate | 99.8: 0.1: 0.1 | 89.05 | 97.96 |
| 40 | Phillygenin/lianqiaoxinside/phillygenin glucuronate | 99.8: 0.1: 0.1 | 82.87 | 91.96 |

**2 In-vivo antiviral test**

**2.1 Experimental materials**

**(1) Experimental animals**

Kunming mice were provided by the Laboratory Animal Center of Bethune Medical Center, Jilin University.

**(2) Detection instruments and reagents**

| Instrument name | Model | Manufacturer |
|---|---|---|
| Quantitative PCR Instrument | 7300 | ABI |
| PCR Instrument | ES-60J | Shenyang Longteng Electronic Weighing Instrument Co., Ltd |
| Electronic Analytical Balance | FA1004 | Shenyang Longteng Electronic Weighing Instrument Co., Ltd |
| CO2 Incubator | HG303-5 | Nanjing Experimental Instrument Factory |
| Superclean bench | SW-CJ-IF | Suzhou Antai AIRTECH Co., Ltd |
| Inverted Microscope | CKX41 | Olympus Instrument |
| -80°C Ultra-low Temperature Freezer | TECON-5082 | Australia |
| Water Bath Shaker | HZS-H | Harbin Donglian Electronic Technology Development Co., Ltd |
| Microplate Reader | TECAN A-5082 | Australia |
| Spectrophotometer | 7550 type | Japan |

**2.2 Experimental method**

**(1) Determination of median lethal doses of influenza virus and parainfluenza virus in mice**

Influenza virus and parainfluenza virus (cell lysate) were diluted in 10-fold into virus solutions having concentrations of $10^{-1}$, $10^{-2}$, $10^{-3}$, $10^{-4}$ and $10^{-5}$. A total of 120 Kunming mice, including 60 mice in a influenza virus group and 60 mice in a parainfluenza virus group, were randomly divided into 6 groups, which were mildly anesthetized with ether, and infected with different dilutions of virus solutions at 0.03 mL/mouse by nasal dripping. Meanwhile, a blank control was provided, and normal saline was used instead of the virus suspension. Death and survival were regarded as observation indexes daily until 14 days after infection. Death within 24 h of infection was non-specific death which was

not counted, and the LD50 of the virus solution was calculated by a Karber method. Calculation formula: $\mathrm{LogLD_{50}=XM+\frac{1}{2}d-d\frac{\sum Pi}{100}}$ [$LD_{50}$: median lethal dose; XM: logarithm of the highest concentration dilution of the virus; d: logarithm of dilution factor (fold); Σpi: a sum of the percentages of lesions per dilution].

**(2) Study of phillyrin/phillygenin composition against pneumonia caused by infections with influenza virus and parainfluenza virus**

**1) Test animals and groups**

Four-week-old Kunming mice were randomly divided into groups (10 mice in each group) and used for a determination test for lung indexes and lung index inhibition rates of a bisepoxylignan compound and composition on mice infected with influenza and parainfluenza viruses.

Further mice were randomly divided into groups (10 mice in each group) and used for a determination test for hemagglutination titers of the bisepoxylignan compound and composition against lung suspension virus.

**2) Infection method**

A lump of absorbent cotton was put into a beaker of 200-300 mL, and then poured with an appropriate amount of ether (till the absorbent cotton was wetted). The beaker filled with the absorbent cotton was inverted, and added with the mice for anesthesia. When the mice were extremely excited and then obviously weak, the mice were taken out with the head facing upward, and then infected with $15LD_{50}$ influenza virus and parainfluenza virus by nasal dripping at 0.03 mL/nostril. A normal control group was administrated with normal saline, instead of a virus suspension.

**(3) Administration method and administration dose**

A bisepoxylignan compound and composition drug group and an Oseltamivir phosphate control group were administrated by routine gavage on one day before infection, respectively; the bisepoxylignan compound and composition drug group was divided into a high-dose group and a low-dose group; the administration doses of the high-dose group and the low-dose group were 13.0 mg/kg and 3.25 mg/kg, respectively, and an administration dose of the Oseltamivir phosphate was 19.5 mg/kg, once a day, for 5 consecutive days; and the normal control group and the virus control group were perfused with the same volume of normal saline.

**4) Observation indexes**

**(1) Lung index determination**

On Day 5 after the administration of each mouse, the mouse was fasted with solids and liquids for 8 h, weighed and then sacrificed by bleeding from the eyeballs; the chest cavity was opened to remove the whole lung which was then washed twice with normal saline; the moisture on the surface was removed by absorption with filter paper; the lung was weighed with an electronic balance; and the lung index and the lung index inhibition rate was calculated according to the following formula:

$$\text{lung index} = (\text{mouse lung weight}/\text{mouse body weight}) \times 100\%$$

lung index inhibition rate = (mean lung index of infection model group - mean lung index of experimental group) / mean lung index of infection model group × 100%

**(2) Determination of hemagglutination titer of lung suspension virus**

The lungs of the mouse in each group on Day 5 after treatment were taken, and ground with a homogenizer at low temperature into a homogenate; the homogenate was diluted with normal saline into a 10% lung tissue suspension; a supernatant was taken by centrifugation, diluted by multiple ratios, and dropped on a titration plate according to 0.2 ml/well; 1% chicken red blood cell suspension was added to each well (0.2 ml/well), mixed well, and placed at room temperature for 30 min; and the hemagglutination titer was observed and recorded. The aggregation of red blood cells (++) was taken as an endpoint, and the titer was expressed by a dilution factor of the suspension.

**2.3 Test results and analysis**

**(1) Determination results of median lethal doses of influenza virus and parainfluenza virus in mice**

The Kunming mice in a test group were respectively infected with 30$\mu$L of influenza virus and parainfluenza virus solutions of different concentrations by nasal dripping, and the mice in three groups (a $10^{-1}$ virus concentrations group, a $10^{-2}$ virus concentration group, and a $10^{-3}$ concentration group) before Day 3 of infection all showed different degrees of symptoms: hair standing, trembling, reduced diet, etc.; on Day 5, the mice showed swaying walking; the mice in a group with a maximum concentration began to die on Day 6; and the rest of the groups died on Day 7 after infection. After 14 days of observation, the number of mice in each group was counted, and the results were shown in the following table. $LD_{50}$ of this influenza virus was calculated as a dilution of $10^{-2.9}$ and that of the parainfluenza virus as $10^{-2.5}$.

**Table 3-4 Statistics of median lethal dose test results of influenza virus**

| Influenza virus group | Cumulative death | Cumulative survival | Cumulative mortality |
|---|---|---|---|
| $10^{-1}$ group | 9 | 1 | 90% |
| $10^{-2}$ group | 7 | 3 | 70% |
| $10^{-3}$ group | 4 | 6 | 40% |
| $10^{-4}$ group | 3 | 7 | 30% |
| $10^{-5}$ group | 1 | 9 | 10% |
| Blank group | 0 | 10 | 0% |

A Karber method was used to calculate $LD_{50}$ of the virus. LogLDso of the influenza virus was as follows:

$$\text{LogLD}_{50}=\text{XM}+\frac{1}{2}\text{d}-\text{d}\frac{\sum Pi}{100}=-1+0.5-(80\%+60\%+40\%+20\%+0\%+0\%)=-2.9$$

**Table 3-5 Statistics of median lethal dose test results of Parainfluenza virus**

| Parainfluenza virus group | Cumulative death | Cumulative survival | Cumulative mortality |
|---|---|---|---|
| $10^{-1}$ group | 8 | 2 | 80% |
| $10^{-2}$ group | 6 | 4 | 60% |
| $10^{-3}$ group | 4 | 6 | 40% |
| $10^{-4}$ group | 2 | 8 | 20% |
| $10^{-5}$ group | 0 | 10 | 0% |
| Blank group | 0 | 10 | 0% |

A Karber method was used to calculate $LD_{50}$ of the virus. LogLDso of parainfluenza virus was as follows:

$$\text{LogLD}_{50}=\text{XM}+\frac{1}{2}\text{d}-\text{d}\frac{\sum Pi}{100}=-1+0.5-(90\%+70\%+40\%+30\%+10\%+0\%)=-2.5$$

**(2) Effect results of phillyrin/phillygenin composition against pneumonia caused by infections with influenza virus and parainfluenza virus**

**(1) Lung index determination**

After the mice were infected with influenza virus and parainfluenza virus, and the mean lung index results showed that compared with an infection model group, the lung index of each of a normal control group, a bisepoxylignan compound and composition drug group and an Oseltamivir phosphate group was significantly reduced (P<0.05 or P<0.01). The high-dose drug group had a lung index lower than that of Oseltamivir phosphate, a lung index inhibition rate higher than that of Oseltamivir phosphate, and efficacy better than that of Oseltamivir phosphate (P<0.01 or P<0.05), and the results were shown in the following table.

**Table 3-6 Lung index inhibition rate of bisepoxylignan compound and composition drug group against mice infected with influenza virus**

| Test group | Division | Lung index ($\overline{X}$ ±S) | Lung index inhibition rate (%) | P-value |
|---|---|---|---|---|
| Normal control group | 0 | 1.172±0.967 | - | |
| Virus control group | 0 | 1.504±0.237 | - | |
| Oseltamivir phosphate group | 19.5 | 1.188±0.156 | 21.01 | ** |
| Lianqiaoxinside. | High dose | 1.169±0.623 | 22.27 | ** |
| | Low dose | 1.185±0.379 | 21.21 | ** |
| Phillyrin | High dose | 1.175±0.259 | 21.88 | * |
| | Low dose | 1.192±0.543 | 20.74 | ** |
| Phillygenin | High dose | 1.177±0.792 | 21.74 | ** |
| | Low dose | 1.196±0.084 | 20.48 | ** |
| Lianqiaoxinside and phillyrin 99.9: 0.1 | High dose | 1.177±0.182 | 21.74 | * |
| | Low dose | 1.183±0.179 | 21.34 | ** |
| Phillyrin and lianqiaoxinside 99.9: 0.1 | High dose | 1.174±0.627 | 21.94 | ** |
| | Low dose | 1.184±0.485 | 21.28 | * |
| Phillygenin and lianqiaoxinside 99.9: 0.1 | High dose | 1.173±0.337 | 22.01 | ** |
| | Low dose | 1.193±0.647 | 20.68 | * |
| Phillyrin/lianqiaoxinside/phillygenin sulfate 99.8: 0.1: 0.1 | High dose | 1.171±0.489 | 22.14 | * |
| | Low dose | 1.183±0.887 | 21.34 | ** |
| Lianqiaoxinside/phillyrin/phillygenin ibuprofenate 99.8: 0.1: 0.1 | High dose | 1.17±0.557 | 22.21 | * |
| | Low dose | 1.191±0.817 | 20.81 | ** |
| Phillygenin/lianqiaoxinside/phillygenin glucuronate 99.8: 0.1: 0.1 | High dose | 1.175±0.213 | 21.88 | ** |
| | Low dose | 1.197±0.879 | 20.41 | ** |
| Each experimental group was compared with the virus control group, where *P<0.05 and **P<0.01. | | | | |

**Table 3-7 Lung index inhibition rates of phillyrin/phillygenin composition against mice infected with parainfluenza virus**

| Test group | Division | Lung index ($\overline{X}$ ±S) | Lung index inhibition rate (%) | P-value |
|---|---|---|---|---|
| Normal control group | 0 | 1.297±0.518 | - | |
| Virus control group | 0 | 1.603±0.724 | - | |
| Oseltamivir phosphate group | 19.5 | 1.315±0.623 | 17.97 | ** |
| Lianqiaoxinside. | High dose | 1.270± 0.863 | 20.77 | ** |
| | Low dose | 1.372± 0.115 | 14.41 | * |
| Phillyrin | High dose | 1.267± 0.015 | 20.96 | * |
| | Low dose | 1.374± 0.848 | 14.29 | ** |

(continued)

| Test group | Division | Lung index ($\overline{X}$ ±S) | Lung index inhibition rate (%) | P-value |
|---|---|---|---|---|
| Phillygenin | High dose | 1.277± 0.056 | 20.34 | ** |
| | Low dose | 1.379± 0.560 | 13.97 | ** |
| Lianqiaoxinside and phillyrin 99.9: 0.1 | High dose | 1.267± 0.833 | 20.96 | * |
| | Low dose | 1.376± 0.478 | 14.16 | ** |
| Phillyrin and lianqiaoxinside 99.9: 0.1 | High dose | 1.266± 0.209 | 21.02 | ** |
| | Low dose | 1.374± 0.426 | 14.29 | ** |
| Phillygenin and lianqiaoxinside 99.9: 0.1 | High dose | 1.269± 0.691 | 20.84 | ** |
| | Low dose | 1.379± 0.441 | 13.97 | * |
| | High dose | 1.268± 0.797 | 20.90 | ** |
| Phillyrin/lianqiaoxinside/phillygenin sulfate 99.8: 0.1: 0.1 | Low dose | 1.374± 0.768 | 14.29 | ** |
| Lianqiaoxinside/phillyrin/phillygenin ibuprofenate 99.8: | High dose | 1.267± 0.125 | 20.96 | * |
| 0.1: 0.1 | Low dose | 1.375± 0.373 | 14.22 | ** |
| Phillygenin/lianqiaoxinside/phillygenin glucuronate 99.8: | High dose | 1.275± 0.293 | 20.46 | ** |
| 0.1: 0.1 | Low dose | 1.378± 0.437 | 14.04 | ** |
| Each experimental group was compared with the virus control group, where *P<0.05 and **P<0.01. | | | | |

**(2) Determination of hemagglutination titer of lung suspension virus**

After the mice were infected with influenza virus and parainfluenza virus, the hemagglutination titers (InX) of lung tissue virus in the infection model group were 35.47 and 34.62, respectively; and the hemagglutination titer of lung tissue virus decreased after 5 days of treatment in the bisepoxylignan compound and composition drug group, and the inhibition rate was significant. Compared with the infection model group, the high- and low-dose groups of the drug group each had the hemagglutination titer lower than that of the Oseltamivir phosphate group, and the inhibition rate significantly higher than that of Oseltamivir phosphate. The test results were shown in the following table.

**Table 3-8 Effect of bisepoxylignan compound and composition on hemagglutination titer in lung suspension of mice infected with influenza virus**

| Test group | Division | Hemagglutination titer | Hemagglutination titer inhibition rate (%) | P-value |
|---|---|---|---|---|
| Normal control group | 0 | | | |
| Virus control group | 0 | 35.47±0.183 | | |
| Oseltamivir phosphate group | 19.5 | 22.07±0.235 | 37.78 | ** |
| Lianqiaoxinside. | High dose | 20.08±0.381 | 43.39 | ** |
| | Low dose | 21.84±0.511 | 38.43 | * |
| Phillyrin | High dose | 20.11±0.351 | 43.30 | * |
| | Low dose | 21.88±0.639 | 38.31 | * |
| Phillygenin | High dose | 20.50±0.489 | 42.20 | * |
| | Low dose | 22.07±0.455 | 37.78 | * |
| Lianqiaoxinside and phillyrin 99.9: 0.1 | High dose | 20.13±0.307 | 43.25 | * |
| | Low dose | 21.81±0.471 | 38.51 | ** |
| Phillyrin and lianqiaoxinside 99.9: 0.1 | High dose | 20.07±0.808 | 43.42 | ** |
| | Low dose | 21.80±0.340 | 38.54 | * |
| Phillygenin and lianqiaoxinside 99.9: 0.1 | High dose | 20.51±0.664 | 42.18 | ** |
| | Low dose | 22.05±0.327 | 37.83 | * |
| Phillyrin/lianqiaoxinside/phillygenin sulfate 99.8: 0.1: 0.1 | High dose | 20.07±0.518 | 43.42 | ** |
| | Low dose | 21.84±0.713 | 38.43 | ** |
| Lianqiaoxinside/phillyrin/phillygenin ibuprofenate 99.8: 0.1: 0.1 | High dose | 20.12±0.528 | 43.28 | * |
| | Low dose | 21.84±0.896 | 38.43 | ** |

(continued)

| Test group | Division | Hemagglutination titer | Hemagglutination titer inhibition rate (%) | P-value |
|---|---|---|---|---|
| Phillygenin/lianqiaoxinside/phillygenin glucuronate 99.8: 0.1: 0.1 | High dose | 20.54±0.834 | 42.09 | ** |
| | Low dose | 22.02±0.058 | 37.92 | ** |
| *P<0.05, **P<0.01. | | | | |

**Table 3-9 Effect of phillyrin/phillygenin composition on hemagglutination titer in lung suspension of mice infected with parainfluenza virus**

| Test group | Division | Hemagglutination titer | Hemagglutination titer inhibition rate (%) | P-value |
|---|---|---|---|---|
| Normal control group | 0 | - | - | |
| Virus control group | 0 | 34.62±0.092 | - | |
| Oseltamivir phosphate group | 19.5 | 23.22±0.648 | 32.93 | * |
| Lianqiaoxinside. | High dose | 21.65±0.238 | 37.46 | ** |
| | Low dose | 23.25±0.847 | 32.84 | * |
| Phillyrin | High dose | 21.63±0.330 | 37.52 | * |
| | Low dose | 23.26±0.219 | 32.81 | ** |
| Phillygenin | High dose | 22.06±0.310 | 36.28 | ** |
| | Low dose | 23.47±0.893 | 32.21 | ** |
| Lianqiaoxinside and phillyrin 99.9: 0.1 | High dose | 21.66±0.645 | 37.44 | ** |
| | Low dose | 23.22±0.512 | 32.93 | ** |
| Phillyrin and lianqiaoxinside 99.9: 0.1 | High dose | 21.64±0.843 | 37.49 | ** |
| | Low dose | 23.26±0.187 | 32.81 | ** |
| Phillygenin and lianqiaoxinside 99.9: 0.1 | High dose | 22.01±0.747 | 36.42 | ** |
| | Low dose | 23.40±0.853 | 32.41 | * |
| Phillyrin/lianqiaoxinside/phillygenin sulfate 99.8: 0.1: 0.1 | High dose | 21.67±0.881 | 37.41 | ** |
| | Low dose | 23.28±0.210 | 32.76 | * |
| Lianqiaoxinside/phillyrin/phillygenin ibuprofenate 99.8: 0.1: 0.1 | High dose | 21.67±0.771 | 37.41 | ** |
| | Low dose | 23.22±0.477 | 32.93 | ** |
| Phillygenin/lianqiaoxinside/phillygenin glucuronate 99.8: 0.1: 0.1 | High dose | 22.03±0.302 | 36.37 | * |
| | Low dose | 23.45±0.364 | 32.26 | * |
| *P<0.05, **P<0.01. | | | | |

**Example 4 Study on in-vivo efficacy of drug of the present invention for treatment of COVID-19 Delta virus**

1.1: Mice: hACE2 mice, aged 6-7 weeks and weighing 20-40 g, laboratory animal supplier: Jiangsu Gempharmatech Co., Ltd., laboratory animal production license: SCXK (Su) 2018-0008, and feed supplier: Jiangsu Medicience Biopharmaceutical Co., Ltd.

1.2 Virus: COVID-19 Delta virus (BSL-3 Laboratory, Guangzhou Customs Technology Center (Laboratory of Highly Pathogenic Microorganisms, State Key Laboratory of Respiratory Diseases).

(1.3) Drugs

Bisepoxylignan compounds: lianqiaoxinside (a purity of 99.95%), phillyrin (content of 99.96%), and phillygenin (a purity of 99.96%); a bisepoxylignan composition: lianqiaoxinside/phillyrin, phillyrin/lianqiaoxinside, phillygenin/lianqiaoxinside, phillyrin/lianqiaoxinside/phillygenin sulfate, lianqiaoxinside/phillyrin/phillygenin ibuprofenate, and phillygeninllianqiaoxinside/phillygenin glucuronate. The above test drugs were provided by Dalian Fusheng Natural Medicine Development Co., Ltd. The above drugs were provided by Dalian Fusheng Natural Medicine Development Co., Ltd.

Remdesivir: a specification of 1 g. Preparation method: during an in-vitro test, DMSO was prepared into mother liquor which was aliquoted, stored at 4°C for later use and diluted to a required concentration with a cell working solution

before use. During an in-vivo test, 0.5% sodium carboxymethyl cellulose was prepared into a required concentration.

1.4 Experimental method

1.4.1 hACE2 transgenic C57BL/6 mice were divided into a normal group, a COVID-19 Delta virus infection group, drug groups (a 40mg/kg low-dose group, and a 80mg/kg high-dose group), and a positive control group (Remdesivir, 50 mg/kg), with eight mice in each group. Except for the mice in the normal group who were administrated with PBS by nasal dripping, the mice in the other groups were infected with COVID-19 Delta virus in $10^5$ PFU by nasal dripping. 2 h after infection, the mice in each drug group were administrated with the drug by gavage for 5 consecutive days, once a day. Dying or dead animals were dissected and lungs were picked after euthanasia; and surviving animals were dissected on the Day 5 after infection, and lungs were picked.

1.4.2 In the infection group, four animals in each group were dissected for lung tissues for pathological study. Four animals were dissected, and lung tissues were picked. Total RNA was extracted from a homogenate supernatant of the lung tissues by a Trizol method, and inflammatory factor interleukin 1β (IL-1β) and monocyte chemoattractant protein 1 (MCP-1) were detected by RT-qPCR.

1.4.3 The specific operation steps of histopathological study of lung tissues were as follows:

(1) fixation and embedding: fixation (fixed with 4% paraformaldehyde) → washing and trimming, trimming tissue to obtain a flat section→ dehydration with 50%, 70%, 80%, 95% and 100% ethanol solutions→ immersion in xylene I and xylene II respectively for transparentizing → immersion with 60°C wax I and II respectively for wax penetration → paraffin embedding; and

(2) slice staining: sectioning (a thickness of about 4 um) → immersion of paraffin section in a clarifying agent I, a clarifying agent II, and a clarifying agent III for deparaffinization → soaking in 100%, 90%, 80%, 70% and other levels of alcohol solutions for 5 min each → washing with water → staining with hematoxylin for 3 min → washing with water → differentiating with 0.5% hydrochloric acid alcohol for a few seconds → washing with tap water for 15 min for bluing → staining with eosin for 2 min → immersion in 95% ethanol I, 95% ethanol II, 100% ethanol I, 100% ethanol II, a clarifying agent I, a clarifying agent II, and a clarifying agent III respectively for dehydration and transparentizing → sealing with neutral gum for storage → observing and photographing under a microscope.

1.4.4 The specific operation steps for the detection of inflammatory factors in mouse lung tissues by RT-qPCR were as follows:

(1) Extraction of RNA

100 mg of mouse lung tissue was placed in a 1.5 mL EP tube, 1 mL of Trizol Reagent was added to the EP tube, the EP tube was inserted into ice, and a grinder was quickly shredded and placed for 5 min at room temperature. The EP tube was added with 200 μL of chloroform, covered tightly, shaken vigorously for 1 min, and set aside at room temperature for 5 min. Centrifugation was performed at 4°C, 10000 rpm/min for 10 min; a suspension was divided into three layers, wherein the upper layer was an aqueous clear liquid; about 500 μL of aqueous phase at the upper layer was pipetted carefully and transferred to a new 1.5 mL EP tube; the EP tube was added with an equal volume of isopropanol, shaken and mixed well, and set aside at room temperature for 10 min. Centrifugation was performed at 4°C, 10000 rpm/min for 10 min. After centrifugation, a milky white translucent gelatinous pellet, i.e., the total RNA, might be seen on a bottom side wall of the EP tube. A supernatant was poured off; 500 μL of 75% ethanol was added to the pellet, cleaned by repeated inversion, and centrifuged at 4°C, 10,000 rpm/min for 10 min. A supernatant was poured off; 500 μL of 75% ethanol was added, cleaned by repeated inversion, and centrifuged at 4°C, 10,000 rpm/min for 5 min. After drying, the ethanol was set aside for 5 min at room temperature, added with RNase-free $ddH_2O$ to dissolve the RNA, and reversely transcribed into cDNA. □

(2) Reverse transcription reaction

The RNA was subjected to a genomic DNA removal reaction before reverse transcription, and the reaction system was shown in the following table:

**Table 4-1 Reaction system table**

| Reagent | Dosage |
|---|---|
| 5×gDNA Eraser Buffer | 2.0 μL |
| gDNA Eraser | 1.0 μL |

(continued)

| Reagent | Dosage |
|---|---|
| Total RNA | 1.0 μL |
| RNase Free $dH_2O$ | up to 10 μL |

Reaction conditions: 42°C, 2 min; RNA from which genomic DNA was removed was stored at 4°C. Reverse transcription of mRNA into cDNA; and a reverse transcription reaction system was configured in the following table:

**Table 4-2 Configured reaction system table**

| Reagent | Dosage |
|---|---|
| The above was a genome-removed RNA reaction solution | 10.0 μL |
| PrimeScript RT Enzyme Mix I | 1.0 μL |
| RT Primer Mix | 1.0 μL |
| 5×PrimeScript Buffer 2 (for Real Time) | 4.0 μL |
| RNase Free $dH_2O$ | 4.0 μL |
| Total | 20.0μL |

Reverse transcription reaction condition were as follows: 37°C, 15 min; 85°C, 5 s; 4°C.

(3) Real-time PCR detection

The reaction system and reaction conditions were as follows:

**Table 4-3 Real-time PCR detection reaction system**

| Reagent | Dosage |
|---|---|
| Premix Ex Taq (2*) | 10.0 μL |
| Upstream primer (20 μM) | 0.5 μL |
| Downstream primer (20 μM) | 0.5 μL |
| Rox Reference Dye II | 0.2 μL |
| Template | 1.0 μL |
| $ddH_2O$ | 7.8 μL |

**Table 4-4 PCR reaction conditions**

| Number of cycles | Steps | Temperature | Time |
|---|---|---|---|
| 1 | Pre-denaturatation | 95°C | 5 min |
| | Denaturation | 95°C | 10 s |
| 40 | Annealing | 55°C | 30 s |
| | Extension | 72°C | 20 s |
| | Solubility curve | 95°C | 1 min |
| 1 | | 55°C | 30 s |
| | | 95°C | 30 s |

(4) Design and synthesis of primers

Primer design software Primer Premier 5.0 was used for primer design, and the primer sequence was as follows:

**Table 4-5 RT-qPCR primer sequence table**

| Target Gene | Direction | Sequence (5'-3') |
|---|---|---|
| IL-1β | Forward | TTGTGTTTGATCTAGAGACCCGCCA |
| | Reverse | TGTGCTGATGCGAGTGCATGATT |
| MCP-1 | Forward | ATCTCAGAAGTGAGGCAGCGTC |
| | Reverse | CGG CA GGTACAGATGGTTGTCAA |

(5) Data statistical processing

Statistical analysis was performed with SPSS23.0 software, which was expressed as mean ± standard deviation ($\bar{x}\pm s$).

1.5. Test results

The pathological results of the lungs of the mice showed that compared with the normal group, the lung tissues of the infection model group presented pathological changes such as pulmonary hemorrhage and interstitial pneumonia. The specific types and degrees of lesions of infected animals in various groups were as follows:

**Table 4-6 Pathomorphological observation results of lungs of mice infected with COVID-19 Delta virus**

| Test group | Division | Pulmonary hemorrhage | | | Interstitial pneumonia | | |
|---|---|---|---|---|---|---|---|
| | | Mild | Moderate | Severe | Mild | Moderate | Severe |
| Virus group | | 0 | 3 | 1 | 0 | 3 | 1 |
| Positive drug group | | 2 | 2 | 0 | 2 | 2 | 0 |
| Lianqiaoxinside. | High dose | 3 | 1 | 0 | 4 | 0 | 0 |
| | Low dose | 2 | 2 | 0 | 2 | 2 | 0 |
| Phillyrin | High dose | 3 | 1 | 0 | 3 | 1 | 0 |
| | Low dose | 2 | 2 | 0 | 2 | 2 | 0 |
| Phillygenin | High dose | 4 | 0 | 0 | 4 | 0 | 0 |
| | Low dose | 2 | 2 | 0 | 2 | 2 | 0 |
| Lianqiaoxinside and phillyrin 99.9: 0.1 | High dose | 4 | 0 | 0 | 4 | 0 | 0 |
| | Low dose | 2 | 2 | 0 | 2 | 2 | 0 |
| Phillyrin and lianqiaoxinside 99.9: 0.1 | High dose | 4 | 0 | 0 | 4 | 0 | 0 |
| Phillygenin and lianqiaoxinside 99.9: | Low dose | 2 | 2 | 0 | 2 | 2 | 0 |
| 0.1 | High dose | 4 | 0 | 0 | 4 | 0 | 0 |
| | Low dose | 2 | 2 | 0 | 2 | 2 | 0 |
| Phillyrin/lianqiaoxinside/phillygenin | High dose | 3 | 1 | 0 | 3 | 1 | 0 |
| sulfate 99.8: 0.1: 0.1 | Low dose | 2 | 2 | 0 | 2 | 2 | 0 |
| Lianqiaoxinside/phillyrin/phillygenin | High dose | 4 | 0 | 0 | 3 | 1 | 0 |
| ibuprofenate 99.8: 0.1: 0.1 | Low dose | 2 | 2 | 0 | 2 | 2 | 0 |
| Phillygenin/lianqiaoxinside/phillygenin | High dose | 4 | 0 | 0 | 4 | 0 | 0 |
| glucuronate 99.8: 0.1: 0.1 | Low dose | 2 | 2 | 0 | 2 | 2 | 0 |

Based on the comparison of the above lesions in various groups, drugs (40 mg/kg and 80 mg/kg) had a significant improvement effect on lung lesions and pulmonary hemorrhage caused by COVID-19 Delta virus, and had pathological improvements of pulmonary hemorrhage and interstitial pneumonia remarkably superior to Remdesivir.

(2) The mRNA expression results of lung inflammatory factors showed that on the Day 5 of COVID-19 Delta virus infection for mice, the expressions of IL-1β and MCP-1 in a virus model group were significantly higher than that in the normal group; compared with the virus model group, IL-1β and MCP-1 inflammatory factors in the high- and low-dose groups (40 mg/kg and 80 mg/kg) were significantly reduced; and the ability to inhibit the overexpression of inflammatory mediators in an 80mg/kg administration group was superior to that of Remdesivir.

**Table 4-7 Expression levels of IL-1β and MCP-1 in lung homogenate of mouse infected with COVID-19 Delta virus ($\bar{x}\pm s$)**

| Drug group | Division | IL-1β | MCP-1 |
|---|---|---|---|
| NC | | 1.00±0.000 | 1.00±0.00 |
| Virus | | 40.93±10.47 | 13.22±7.96 |
| Remdesivir | 50mg/kg | 9.52±6.37 | 6.88±5.73 |
| Lianqiaoxinside. | High-dose group | 4.80±18.82 | 3.26±9.77 |
| | Low-dose group | 9.13±8.66 | 6.13±15.12 |
| Phillyrin | High-dose group | 5.11±14.34 | 3.66±11.30 |
| | Low-dose group | 10.58±10.22 | 7.42±12.66 |
| Phillygenin | High-dose group | 4.93±16.18 | 3.67±10.22 |
| | Low-dose group | 9.98±11.16 | 6.71±4.38 |
| Lianqiaoxinside and phillyrin 99.9: 0.1 | High-dose group | 4.41±6.14 | 3.34±10.93 |
| | Low-dose group | 9.08±5.96 | 6.68±8.18 |
| Phillyrin and lianqiaoxinside 99.9: 0.1 | High-dose group | 5.04±8.13 | 3.73±2.98 |
| | Low-dose group | 10.53±3.87 | 7.59±9.96 |
| Phillygenin and lianqiaoxinside 99.9: 0.1 | High-dose group | 4.73±17.14 | 3.65±17.51 |
| | Low-dose group | 9.85±6.27 | 6.98±6.53 |
| Phillyrinllianqiaoxinside/phillygenin sulfate 99.8: 0.1: 0.1 | High-dose group | 5.25±18.63 | 4.07±11.97 |
| | Low-dose group | 10.67±9.54 | 7.94±19.82 |
| Lianqiaoxinside/phillyrin/phillygenin ibuprofenate 99.8: 0.1: 0.1 | High-dose group | 4.58±9.05 | 3.61±11.34 |
| | Low-dose group | 9.20±7.39 | 6.64±10.06 |
| Phillygenin/lianqiaoxinside/phillygenin glucuronate 99.8: 0.1: 0.1 | High-dose group | 4.71±7.42 | 3.71±12.10 |
| | Low-dose group | 9.83±15.93 | 7.43±7.34 |

**Example 5 Study on in-vivo efficacy of drug of the present invention for treatment of influenza virus**

1.1 Mice: ICR mice, weighing 20-30 g, laboratory animal supplier: Hunan SJA Laboratory Animal Co., Ltd., laboratory animal production license: SCXK (Xiang) 2019-0004, and feed supplier: Jiangsu Medicience Biopharmaceutical Co., Ltd.

1.2 Virus: influenza virus (BSL-3 Laboratory, Guangzhou Customs Technology Center (Laboratory of Highly Pathogenic Microorganisms, State Key Laboratory of Respiratory Diseases).

(1.3) Drugs

Bisepoxylignan compounds: lianqiaoxinside (a purity of 99.95%), phillyrin (content of 99.96%), and phillygenin (a purity of 99.96%); and bisepoxylignan compositions: lianqiaoxinside/phillyrin, phillyrin/lianqiaoxinside, phillygenin/lianqiaoxinside, phillyrin/lianqiaoxinside/phillygenin sulfate, lianqiaoxinside/phillyrin/phillygenin ibuprofenate, and phillygeninllianqiaoxinside/phillygenin glucuronate. The above test drugs were provided by Dalian Fusheng Natural Medicine Development Co., Ltd.

Oseltamivir phosphate: China Institute for the Control of Pharmaceutical and Biological Products. Product batch number: 101096-201901, 100 mg/piece as a positive control drug in this test.

1.4 Experimental method

(1) Virus inoculation

Influenza virus intranasal vaccination: a lump of absorbent cotton was put into a beaker of 200-300 mL, and then poured with an appropriate amount of ether (till the absorbent cotton was wetted). The beaker filled with the absorbent cotton was inverted, and added with the mice for anesthesia. When the mice were extremely excited and then obviously weak, that is, the anesthesia depth was moderate, the mice were taken out. The ether-anesthetized mice were placed on their backs, with the mouse head facing upward, and then were subjected to intranasal infection. A diluted influenza virus was added to nostrils at 0.03 mL/nostril ($15LD_{50}$). A blank control was administrated with normal saline, instead

of a virus suspension.

(2) Administration method

A bisepoxylignan composition and composition drug groups (high dose of 26.0 mg/kg, low dose of 13.0 mg/kg), a model group and an Oseltamivir phosphate drug control group were administrated by routine gavage on one day before infection, once a day, for 5 consecutive days; and the positive blank group and the model group were perfused with the same volume of 0.5% sodium carboxymethylcellulose.

(3) IL-2 and TNF-a detection

On the Day 5 after influenza virus infection, that is, Day 6 after administration, after the mice were fasted with solids for 8 h and weighed, venous blood was collected from the plexus ophthalmicus, and blood from mice in each group was preserved, subjected to serum separation, and stored in a refrigerator for later use. According to the instructions of a test kit, various detection and analysis were carried out, and IL-2 and TNF-a concentrations were calculated based on the standard detection and standard curve plotting.

(4) TNF-a and IL-2 detection

(2) The results showed that different concentrations of drugs intervened in both TNF-a and IL-2. The efficacy of the high-dose group was better than that of Oseltamivir phosphate.

**Table 5-1 Expression levels of TNF-a and IL-2 in mice infected with influenza virus ($\bar{x}\pm s$)**

| Division | Dose | TNF-a | IL-2 |
|---|---|---|---|
| NC | | 1056.39±32.84 | 150.73± 18.65 |
| Virus | | 808.34±20.44 | 291.12±19.33 279.83± |
| Oseltamivir phosphate | 19.5mg/kg | 658.91±38.33 | 23.26 |
| Lianqiaoxinside. | High-dose group | 608.95±34.92 | 259.04±39.98 |
| | Low-dose group | 673.65±26.44 | 288.64±25.05 |
| Phillyrin | High-dose group | 618.38±28.86 | 262.88±35.66 |
| | Low-dose group | 683.47±37.37 | 293.00±21.88 |
| Phillygenin | High-dose group | 648.40±27.19 | 275.59±31.88 |
| | Low-dose group | 713.48±39.74 | 306.70±34.88 |
| Lianqiaoxinside and phillyrin 99.9: 0.1 | High-dose group | 608.15±20.32 | 258.77±38.58 |
| | Low-dose group | 672.28±36.36 | 289.20±30.21 |
| Phillygenin and lianqiaoxinside 99.9: 0.1 | High-dose group | 618.12±37.74 | 263.36±38.73 |
| | Low-dose group | 683.65±38.70 | 293.17±20.80 |
| Phillyrin/lianqiaoxinside/phillygenin sulfate 99.8: 0.1: 0.1 | High-dose group | 648.89±38.16 | 275.87±21.29 |
| | Low-dose group | 713.00±29.96 | 305.89±31.11 |
| Lianqiaoxinside/phillyrin/phillygenin ibuprofenate 99.8: 0.1: 0.1 | High-dose group | 618.22±29.63 | 263.25±38.96 |
| | Low-dose group | 683.95±34.43 | 293.02±34.68 |
| Lianqiaoxinside/phillyrin/phillygenin ibuprofenate 99.8: 0.1: 0.1 | High-dose group | 608.79±39.70 | 258.57±39.22 |
| | Low-dose group | 673.91±36.51 | 288.55±25.86 |
| Phillygenin/lianqiaoxinside/phillygenin glucuronate 99.8: 0.1: 0.1 | High-dose group | 648.94±23.00 | 276.40±20.88 |
| | Low-dose group | 713.39±36.17 | 301.22±34.23 |

(5) Histological and pathological examination of lung lesions

4 mouse lungs in various experimental groups were taken for fixation, embedding, section staining, microscopic examination, and the like. The pathological results of the lungs of the mice showed that compared with the normal group, the lung tissues of the infection model group presented pathological changes such as pulmonary hemorrhage and interstitial pneumonia. The specific types and degrees of lesions of infected animals in various groups were as follows:

**Table 5-2 Pathomorphological observation results of lungs of mice infected with influenza virus**

| Test group | Division | Pulmonary hemorrhage | | | Interstitial pneumonia | | |
|---|---|---|---|---|---|---|---|
| | | Mild | Moderate | Severe | Mild | Moderate | Severe |
| Virus group | | 0 | 4 | 0 | 0 | 4 | 0 |
| Positive drug group | | 2 | 2 | 0 | 2 | 2 | 0 |
| Lianqiaoxinside. | High dose | 4 | 0 | 0 | 4 | 0 | 0 |
| | Low dose | 2 | 2 | 0 | 2 | 2 | 0 |
| Phillyrin | High dose | 4 | 0 | 0 | 4 | 0 | 0 |
| | Low dose | 2 | 2 | 0 | 2 | 2 | 0 |
| Phillygenin | High dose | 3 | 1 | 0 | 3 | 1 | 0 |
| | Low dose | 2 | 2 | 0 | 2 | 2 | 0 |
| Lianqiaoxinside and phillyrin 99.9: 0.1 | High dose | 4 | 0 | 0 | 4 | 0 | 0 |
| | Low dose | 2 | 2 | 0 | 2 | 2 | 0 |
| Phillyrin and lianqiaoxinside 99.9: 0.1 | High dose | 4 | 0 | 0 | 4 | 0 | 0 |
| | Low dose | 2 | 2 | 0 | 2 | 2 | 0 |
| Phillygenin and lianqiaoxinside 99.9: 0.1 | High dose | 3 | 1 | 0 | 3 | 1 | 0 |
| | Low dose | 2 | 2 | 0 | 2 | 2 | 0 |
| Phillyrin/lianqiaoxinside/phillygenin sulfate 99.8: 0.1: 0.1 | High dose | 4 | 0 | 0 | 4 | 0 | 0 |
| | Low dose | 2 | 2 | 0 | 2 | 2 | 0 |
| Lianqiaoxinside/phillyrin/phillygenin ibuprofenate 99.8: 0.1: 0.1 | High dose | 4 | 0 | 0 | 4 | 0 | 0 |
| | Low dose | 2 | 2 | 0 | 2 | 2 | 0 |
| Phillygenin/lianqiaoxinside/phillygenin glucuronate 99.8: 0.1: 0.1 | High dose | 3 | 1 | 0 | 3 | 1 | 0 |
| | Low dose | 2 | 2 | 0 | 2 | 2 | 0 |

Based on the comparison of lesions in various groups, the pathological changes of the surviving animals in the various groups were milder than dead animals; lesion degrees of the drug low-dose group and the drug high-dose group were milder than those of the virus group, and drugs (13.0 mg/kg and 26 mg/kg) had good improvement effects on lung lesions caused by influenza virus and had an effect better than Oseltamivir phosphate.

**Example 6 Study on the efficacy of the drug of the present invention for the prevention of influenza virus**

1. Drugs

Bisepoxylignan compounds include: lianqiaoxinside (having a purity of 99.95%), phillyrin (having a purity of 99.96%), phillygenin (having a purity of 99.96%), phillygenin sulfate (having a purity of 99.95%), phillygenin glucuronate (having a purity of 99.95%), or phillygenin ibuprofenate (having a purity of 99.95%).

Bisepoxylignan compositions include: lianqiaoxinside/phillyrin, lianqiaoxinside/phillygenin, lianqiaoxinside/phillygenin sulfate, lianqiaoxinside/phillygenin glucuronate, lianqiaoxinside/phillygenin ibuprofenate, phillyrin/phillygenin sulfate, phillyrin/phillygenin glucuronate, phillyrin/phillygenin ibuprofenate, phillygenin/phillygenin sulfate, phillygenin/phillygenin glucuronate, phillygenin/phillygenin ibuprofenate, phillygenin sulfate/phillygenin glucuronate, phillygenin sulfate/phillygenin ibuprofenate, phillygenin glucuronate/phillygenin ibuprofenate, phillyrin/lianqiaoxinside/phillygenin sulfate, lianqiaoxinside/phillyrin/phillygenin ibuprofenate, and phillygeninllianqiaoxinside/phillygenin glucuronate.

The above test drugs were provided by Dalian Fusheng Natural Medicine Development Co., Ltd.

(2) Cell line: Vero E6 cells (African green monkey kidney cells), State Key Laboratory of Respiratory Diseases, Guangzhou Institute of Respiratory Medicine.

(3) Virus strain: influenza virus FM1 strains of influenza virus and parainfluenza virus: Institute of Virology, Chinese Academy of Preventive Medicine.

2. The specific action links of the drugs were discussed, and an administration method and a test purpose were as follows:

Group I: a virus solution was added after 24 h of drug administration; whether the drug could enter the cell or be adsorbed on the cell surface to prevent the adsorption and entry of the virus and to play a preventive role in the virus was observed, which is equivalent to administrating the drug first and then infecting the influenza virus; and the efficacy of the drug in preventing influenza was observed.

Group II: a virus solution was added first, a drug was added 1 h after adsorption, and whether the drug can work on the virus entering the cell, inhibit its biosynthesis and mature release and play a therapeutic role was observed, which was equivalent to being infected with the influenza virus before administration, and observing the therapeutic effect.

Group III: a drug solution and a virus solution were added at the same time, the effects of the drug and the virus under different conditions were observed, and whether the drug had a direct inactivation effect on the virus was observed, which was equivalent to immediate administration after infection with influenza, and observation of the therapeutic effect.

Group IV: a drug solution and a virus solution were mixed and adsorbed for 2 h and then added with cells, and the effects of the drug and the virus under different conditions and whether the drug had a direct inactivation effect on the virus were observed, which was equivalent to administration after infection with influenza, and observation of the therapeutic effect.

**3. Experimental results**

Group I: the test results showed that the bisepoxylignan compound and composition could enter the cell or be adsorbed on the cell surface, prevent the adsorption and entry of influenza virus and parainfluenza virus, with a blocking effect of more than 75%.

Group II: the test results showed that the bisepoxylignan compound and composition could play a role in the virus entering the cell, and had an effect of inhibiting the synthesis and release of influenza virus and parainfluenza virus, with an inhibition rate of more than 75%.

Groups III and IV: the test results showed that the bisepoxylignan compound and composition had a direct inactivation effect on influenza virus and parainfluenza virus. A bisepoxylignan compound and composition solution and a virus solution were added at the same time, and an inactivation rate of influenza and parainfluenza viruses reached about 100%. The bisepoxylignan compound and composition solution and the virus were mixed and adsorbed for 2 h, and then added with cells, with inactivation rates for the parainfluenza virus of about 100%. Therefore, regardless of the length of time for the virus to interact with the bisepoxylignan compound and composition, as long as the bisepoxylignan compound and composition had the opportunity to be in direct contact with the above-mentioned viruses, they had obvious inactivation effects, and also had effects significantly better than those of other pathways of action. Details were shown in the following Table.

**Table 6-1 Comparison of protection rates of phillyrin against virus-infected tissue cells (ER%)**

| Tested drug | Virus | Protection rate | | | |
|---|---|---|---|---|---|
| | | Group I | Group II | Group III | Group IV |
| Lianqiaoxinside. | Influenza | 75.49 | 75.77 | 100.52 | 100.76 |
| | Parainfluenza | 84.39 | 85.95 | 100.49 | 100.82 |
| Phillyrin | Influenza | 75.86 | 75.58 | 100.68 | 100.86 |
| | Parainfluenza | 84.48 | 85.94 | 100.67 | 100.93 |
| Phillygenin | Influenza | 75.46 | 75.51 | 100.86 | 100.38 |
| | Parainfluenza | 84.15 | 85.11 | 100.46 | 100.18 |
| Lianqiaoxinside and phillyrin 99.9: 0.1 | Influenza | 75.63 | 75.37 | 100.52 | 100.44 |
| | Parainfluenza | 84.75 | 85.57 | 100.13 | 100.30 |
| Phillyrin and lianqiaoxinside 99.9: 0.1 | Influenza | 75.22 | 75.96 | 100.40 | 100.47 |
| | Parainfluenza | 84.98 | 85.45 | 100.16 | 100.31 |
| Phillygenin and lianqiaoxinside 99.9: 0.1 | Influenza | 75.43 | 75.15 | 100.51 | 100.94 |
| | Parainfluenza | 84.95 | 85.89 | 100.65 | 100.41 |
| Phillyrin/lianqiaoxinside/phillygenin sulfate 99.8: 0.1: 0.1 | Influenza | 75.51 | 75.26 | 100.40 | 100.26 |
| | Parainfluenza | 84.23 | 85.19 | 100.85 | 100.86 |
| Lianqiaoxinside/phillyrin/phillygenin ibuprofenate 99.8: 0.1: 0.1 | Influenza | 75.84 | 75.59 | 100.93 | 100.42 |
| | Parainfluenza | 84.34 | 85.32 | 100.49 | 100.86 |

(continued)

| Tested drug | Virus | Protection rate | | | |
|---|---|---|---|---|---|
| | | Group I | Group II | Group III | Group IV |
| Phillygenin/lianqiaoxinside/phillygenin glucuronate 99.8: 0.1: 0.1 | Influenza | 75.42 | 75.82 | 100.21 | 100.50 |
| | Parainfluenza | 84.57 | 85.31 | 100.20 | 100.25 |

**Example 7 Study on efficacy of drug of the present invention for the prevention of COVID-19 Delta virus**

1.1 Mice: hACE2 male mice, aged 6-7 weeks and weighing 20-40 g, laboratory animal supplier: Jiangsu Gempharmatech Co., Ltd., laboratory animal production license: SCXK (Su) 2018-0008, and feed supplier: Jiangsu Medicience Biopharmaceutical Co., Ltd.

1.2 COVID-19 Delta virus (P3 Laboratory, Guangzhou Customs Technology Center (Laboratory of Highly Pathogenic Microorganisms, State Key Laboratory of Respiratory Diseases)

(1.3) Drugs

Bisepoxylignan compounds: lianqiaoxinside (a purity of 99.95%), phillyrin (content of 99.96%), and phillygenin (a purity of 99.96%); bisepoxylignan compositions: lianqiaoxinside/phillyrin, phillyrin/lianqiaoxinside, phillygenin/lianqiaoxinside, phillyrin/lianqiaoxinside/phillygenin sulfate, lianqiaoxinside/phillyrin/phillygenin ibuprofenate, and phillygeninllianqiaoxinside/phillygenin glucuronate. The above test drugs were provided by Dalian Fusheng Natural Medicine Development Co., Ltd.

Remdesivir: a specification of 1 g. Preparation method: during an in-vitro test, DMSO was prepared into mother liquor which was aliquoted, stored at 4°C for later use and diluted to a required concentration with a cell working solution before use. During an in-vivo test, 0.5% sodium carboxymethyl cellulose was prepared into a required concentration.

1.4 Experimental method

hACE2 transgenic C57BL/6 mice were divided (8 mice in each group) into a normal group (NC), a virus infection group (Virus), and drug groups (a COVID-19 Delta virus low-dose (40 mg/kg) group, high-dose (80 mg/kg) group; and a positive control group (Remdesivir 50 mg/kg). Before nasal dripping infection, the drug was administered by gavage 5 days in advance, once a day. After 5 days of continuous gavage, nasal dripping infection was performed, and mice in each COVID-19 virus infection group were infected with COVID-19 Delta virus with $10^5$ PFU by nasal dripping, except for the normal group who were given PBS by nasal dripping.

After infection, the body weight change was recorded every day, the death of animals within 5 days of infection was recorded, and the mean survival days and mortality rate of 5 days were calculated. On the Day 5 after infection, the lungs were dissected, and half of the lung tissues was homogenized for virus titer detection.

The virus titer detection for mouse lung homogenate included: picking lung tissues of mice, placing in a petri dish, shredding and transferring into a homogenization tube, diluting with normal saline 1:10 (w/v), and homogenizing at 8000 rpm/min for 10 min. The above operations were all done in an ice bath. The homogenate was transferred to a 1.5 mL EP tube and centrifuged at 4°C, 10,000 rpm for 10 min. The supernatant was pipetted, aliquoted, and stored at -80 °C for later use.

VERO E6 cells with good growth status were inoculated in a 96-well plate at $1\times10^4$ cells/well, and continued to be cultured for 24 h. After the cells grew into a complete monolayer adherently, a supernatant was discarded and the cells were washed twice in PBS. The cryopreserved lung homogenate supernatant was thawed, and then diluted to five concentrations of $10^{-1}$ to $10^{-5}$ in a 10-fold ratio; the diluted lung homogenate supernatant of the mouse in each group was added in a 96-well plate; 100 μL of lung supernatant was added to each well; blank control wells are set at the same time; a cell culture medium was added to the blank control wells; and 8 duplicate wells were made in parallel for each concentration and cultured in the incubator. A cytopathic effect (CPE) was observed daily for 4 consecutive days, the number of lesion wells at each gradient concentration was recorded, and its TCID50 value for VERO E6 cells was calculated.

1.5 Experimental results

(1) The results of protective study on prophylactic administration of drug of the present invention on the death caused by COVID-19 virus

The specific data was shown in Table 14. 100% of mice died in the virus group, the death protection rate of the mouse in a Remdesivir administration group was 75%, and the drug of the present invention had a protective effect of up to 87.50% on the mouse infected with the COVID-19 virus; and the drug group of the present invention with prophylactic administration had a higher death protection rate than the Remdesivir group.

The prophylactic administration of the drug of the present invention had a virus titer for the pneumovirus of mice infected with COVID-19 Delta virus.

The results of viral titer detection were shown in the following table. The results showed that the pneumovirus titer of mice in the drug administration group of the present invention was significantly reduced.

**Table 7-1 Experimental results of prophylactic administration on the death protection of Delta-infected mice ($\bar{x}\pm s$, n=8)**

| Drug name | Division | Mean survival | Survival rate | Death rate |
|---|---|---|---|---|
| NC | -- | 5.00±0.00 | 100 | 0.00 |
| Virus | -- | 2.63±0.52 | 0 | 100.00 |
| Remdesivir-50mg/kg | -- | 3.88±0.83 | 25.00% | 75.00% |
| Lianqiaoxinside. | High dose | 4.88±0.35 | 87.50% | 12.50% |
| | Low dose | 4.25±0.46 | 25.00% | 75.00% |
| Phillyrin | High dose | 4.88±0.35 | 87.50% | 12.50% |
| | Low dose | 3.88±0.83 | 25.00% | 75.00% |
| Phillygenin | High dose | 4.88±0.35 | 87.50% | 12.50% |
| | Low dose | 4.13±0.64 | 25.00% | 75.00% |
| Lianqiaoxinside and phillyrin 99.9: 0.1 | High dose | 4.88±0.35 | 87.50% | 12.50% |
| | Low dose | 4.25±0.46 | 25.00% | 75.00% |
| Phillyrin and lianqiaoxinside 99.9: 0.1 | High dose | 4.88±0.35 | 87.50% | 12.50% |
| | Low dose | 4.00±0.76 | 25.00% | 75.00% |
| Phillygenin and lianqiaoxinside 99.9: 0.1 | High dose | 4.88±0.35 | 87.50% | 12.50% |
| | Low dose | 4.00±0.76 | 25.00% | 75.00% |
| Phillyrin/lianqiaoxinside/phillygenin sulfate 99.8: 0.1: 0.1 | High dose | 4.88±0.35 | 87.50% | 12.50% |
| | Low dose | 3.88±0.83 | 25.00% | 75.00% |
| Lianqiaoxinside/phillyrin/phillygenin ibuprofenate 99.8: 0.1: 0.1 | High dose | 4.88±0.35 | 87.50% | 12.50% |
| | Low dose | 4.25±0.46 | 25.00% | 75.00% |
| Phillygenin/lianqiaoxinside/phillygenin glucuronate 99.8: 0.1: 0.1 | High dose | 4.88±0.35 | 87.50% | 12.50% |
| | Low dose | 4.13±0.64 | 25.00% | 75.00% |

**Table 7-2 Detection results of virus titer in lung tissues of mice infected with COVID-19 Delta ($\bar{x}\pm s$, n=8)**

| Groups | Division | Lung virus titer |
|---|---|---|
| NC | -- | - |
| Virus | -- | 3.83±0.91 |
| Remdesivir-50mg/kg | -- | 3.69±1.06 |
| Lianqiaoxinside. | High dose | 2.93±0.34 |
| | Low dose | 3.36±0.28 |
| Phillyrin | High dose | 2.95±0.57 |
| | Low dose | 3.70±0.25 |
| Phillygenin | High dose | 2.98±0.73 |
| | Low dose | 3.47±0.98 |

(continued)

| Groups | Division | Lung virus titer |
|---|---|---|
| Lianqiaoxinside and phillyrin 99.9: 0.1 | High dose | 2.91±0.35 |
| | Low dose | 3.35±1.33 |
| Phillyrin and lianqiaoxinside 99.9: 0.1 | High dose | 2.95±0.35 |
| | Low dose | 3.58±0.79 |
| Phillygenin and lianqiaoxinside 99.9: 0.1 | High dose | 2.97±1.00 |
| | Low dose | 3.57±1.23 |
| Phillyrin/lianqiaoxinside/phillygenin sulfate 99.8: 0.1: 0.1 | High dose | 2.90±0.52 |
| | Low dose | 3.68±0.40 |
| Lianqiaoxinside/phillyrin/phillygenin ibuprofenate 99.8: 0.1: 0.1 | High dose | 2.94±0.54 |
| | Low dose | 3.37±0.49 |
| Phillygenin/lianqiaoxinside/phillygenin glucuronate 99.8: 0.1: 0.1 | High dose | 2.95±0.38 |
| | Low dose | 3.46±1.49 |

## Claims

1. An application of a bisepoxylignan compound or a bisepoxylignan composition in the preparation of health food or drug for prevention or treatment of infections with influenza viruses, parainfluenza viruses, COVID-19 viruses or COVID-19 virus variants.

2. An application of a bisepoxylignan compound or a bisepoxylignan composition in the preparation of health food or drug for prevention or treatment of pulmonary hemorrhage caused by COVID-19 viruses or COVID-19 virus variants.

3. An application of a bisepoxylignan compound or a bisepoxylignan composition in the preparation of health food or drug for resisting pneumonia caused by influenza viruses, parainfluenza viruses, COVID-19 viruses and COVID-19 virus variants.

4. The application according to any one of claims 1 to 3, wherein the bisepoxylignan compound refers to a bisepoxylignan monomer compound.

5. The application according to claim 4, wherein the bisepoxylignan compound is phillyrin, lianqiaoxinside, phillygenin, phillygenin sulfate, phillygenin glucuronate or phillygenin ibuprofenate.

6. The application according to any of claims 1 to 3, wherein the bisepoxylignan composition is a composition consisting of phillyrin and one or more of lianqiaoxinside, phillygenin sulfate, phillygenin glucuronate and phillygenin ibuprofenate respectively.

7. The application according to claim 6, wherein the bisepoxylignan composition is a composition consisting of lianqiaoxinside and phillyrin, a composition consisting of phillyrin and phillygenin sulfate, a composition consisting of phillyrin and phillygenin glucuronate, a composition consisting of phillyrin and phillygenin ibuprofenate, a composition consisting of phillyrin, lianqiaoxinside and phillygenin sulfate, or a composition consisting of lianqiaoxinside, phillyrin and phillygenin ibuprofenate.

8. The application according to any of claims 1 to 3, wherein the bisepoxylignan composition is a composition consisting of any two or more of lianqiaoxinside, phillygenin, phillygenin sulfate, phillygenin glucuronate and phillygenin ibuprofenate.

9. The application according to claim 8, wherein the bisepoxylignan composition is a composition consisting of lianqiaoxinside and phillygenin, a composition consisting of lianqiaoxinside and phillygenin sulfate, a composition consisting of lianqiaoxinside and phillygenin glucuronate, a composition consisting of lianqiaoxinside and phillygenin ibuprofenate, a composition consisting of phillygenin and phillygenin sulfate, a composition consisting of phillygenin and phillygenin glucuronate, a composition consisting of phillygenin and phillygenin glucuronate, a composition of phillygenin sulfate and phillygenin glucuronate, a composition consisting of phillygenin sulfate and phillygenin ibuprofenate, a composition consisting of phillygenin glucuronate and phillygenin ibuprofenate, or a composition consisting of phillygenin, lianqiaoxinside and phillygenin glucuronate.

**10.** The application according to any one of claims 1 to 3, wherein the COVID-19 virus variant is a COVID-19 virus variant Beta or a COVID-19 virus variant Delta.

**11.** The application according to any one of claims 1 to 3, wherein the bisepoxylignan compound and the bisepoxylignan composition are applied as a sole active ingredient.

**12.** The application according to any one of claims 1 to 3, wherein the drug is present in the form of tablets, capsules, pills, powders, granules, a syrup, a solution, an emulsion, an injection, a spray, an aerosol, a gel, a cream, cataplasm, a rubber plaster or plaster; or, the health food is present in the food form of tablets, capsules, pills, powders, granules, a syrup, a solution, an emulsion, a spray, an aerosol, a gel, a cream, cataplasm, a rubber plaster or plaster, candies and preserved fruits, tea, a beverage, baked food or a wine product.

**13.** A synthesis method of lianqiaoxinside, comprising the following steps:

1) obtaining corresponding β-keto esters (a compound of formula 4) and (a compound of formula 9) by taking vanillin (a compound of formula 1) and 3-hydroxy-5 methoxybenzaldehyde (a compound of formula 6) as raw materials via phenolic hydroxyl protection, oxidization and Claisen condensation reaction; then converting the β-keto esters into corresponding sodium salt (a compound of formula 5) and bromide (a compound of formula 10) respectively, and then performing a coupling reaction on the two fragments to obtain an intermediate (a compound of formula 11);
2) obtaining aglycone (a compound of formula 14) from the intermediate (the compound of formula 11) via a reduction reaction and a ring-closing reaction;
3) obtaining a target product (a compound of formula 17) from the aglycone (the compound of formula 14) via glycosylation and protecting group removal; and

a reaction formula is as follows:

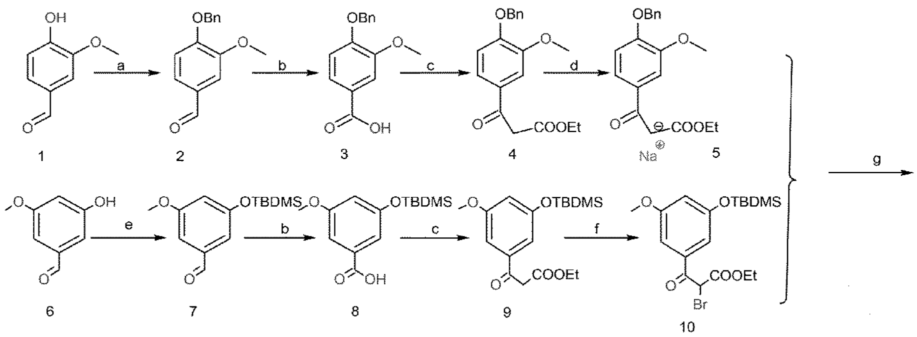

1 2 3 4 5
6 7 8 9 10

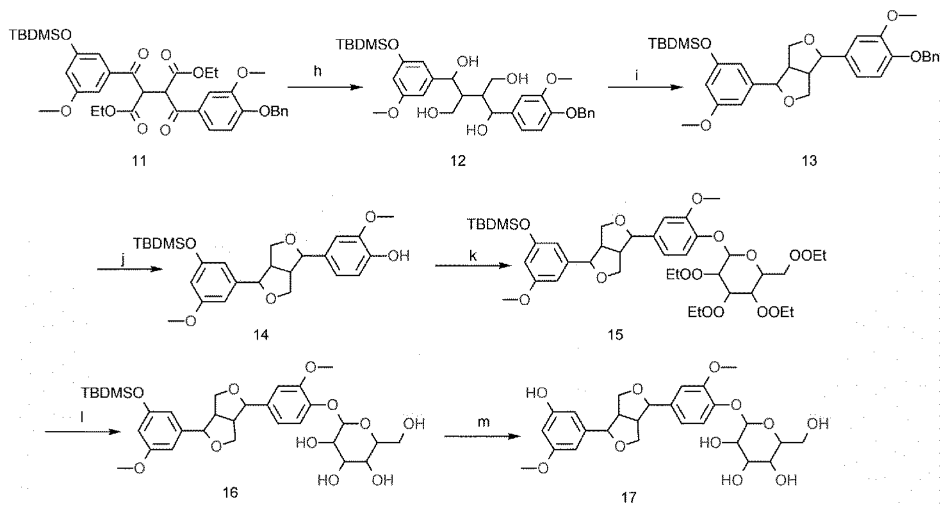


11 12 13 14 15 16 17

in which: a. benzyl chloride; b. potassium permanganate; c. thionyl chloride/ethyl acetoacetate/sodium ethoxide; d. sodium ethoxide; e. tert-butyldimethylchlorosilane; f. bromine water; g. methylene chloride; h. lithium aluminum hydride; i. methylsulfonyl chloride/pyridine; j. palladium carbon/hydrogen; k. acetylglucosyl trichloroethanimidate; l. sodium methoxide; and m. tetrabutylammonium fluoride.

**14.** The synthesis method according to claim 13, wherein in step 1):

synthesis of a compound of formula 2 comprises: dissolving vanillin in acetonitrile, adding potassium carbonate, potassium iodide and benzyl chloride, and performing a reflux reaction for 5 h to obtain the compound of formula 2;
synthesis of a compound of formula 3 comprises: dissolving the compound of formula 2 in hot water, adding potassium permanganate, and reacting at 70-80°C for 1 h to obtain the compound of formula 3;
synthesis of a compound of formula 4 comprises: dissolving the compound of formula 3 in thionyl chloride, reacting at 95°C for 8 h, removing thionyl chloride by distillation, adding anhydrous tetrahydrofuran and a sodium salt of ethyl acetoacetate, refluxing for 8 h, removing tetrahydrofuran, then adding ammonium chloride and 95% ethanol solution containing 1% ammonia water directly to residues, and refluxing for 4 h to obtain the compound of formula 4;
synthesis of a compound of formula 7 comprises: dissolving 3-hydroxy-5 methoxybenzaldehyde in N,N-dimethylformamide, adding imidazole and tert-butyldimethylchlorosilane, and reacting at 35°C for 10 h to obtain the compound of formula 7;
synthesis of a compound of formula 8 comprises: dissolving the compound of formula 7 in hot water, adding potassium permanganate, and reacting at 70-80°C for 1 h to obtain the compound of formula 8;
synthesis of a compound of formula 9 comprises: dissolving the compound of formula 8 in thionyl chloride, reacting at 95°C for 8 h, removing thionyl chloride by distillation, adding anhydrous tetrahydrofuran and a sodium salt of ethyl acetoacetate, refluxing for 8 h, removing tetrahydrofuran, then adding ammonium chloride and 95% ethanol solution containing 1% ammonia water directly to residues, and refluxing for 4 h to obtain the compound of formula 9;
and/or,
synthesis of a compound of formula 11 comprises: adding the compound of formula 4 to an absolute ethanol solution dissolved with the same amount of metal sodium, and stirring and
reacting at room temperature for 3 h to obtain a compound of formula 5; dissolving the compound of formula 9 in an anhydrous tetrahydrofuran solution, adding bromine water, and stirring and
reacting at room temperature for 3 h to obtain a compound of formula 10; and dissolving the compound of formula 5 and the compound of formula 10 in a dichloromethane solution, and
stirring and reacting at room temperature for 10 h to obtain the compound of formula 11.

**15.** The synthesis method according to claim 13, wherein in step 2):

synthesis of a compound of formula 12 comprises: dissolving the compound of formula 11 in anhydrous tetrahydrofuran, adding an anhydrous tetrahydrofuran solution containing lithium aluminum hydride, performing a reflux reaction for 6 h, and stirring at room temperature overnight to obtain the compound of formula 12;

synthesis of a compound of formula 13 comprises: dissolving the compound of formula 12 in re-distilled benzene, adding pyridinium p-toluenesulfonate salt powder, and performing a reflux reaction for 3 h to obtain the compound of formula 13; and/or,

synthesis of a compound of formula 14 comprises: dissolving the compound of formula 13 in methanol under a hydrogen condition, adding 10% palladium carbon, and stirring and reacting at room temperature for 10 h to obtain the compound of formula 14.

**16.** The synthesis method according to claim 13, wherein in step 3):

synthesis of a compound of formula 15 comprises: dissolving the compound of formula 14 in anhydrous dichloromethane under the protection of nitrogen, adding tetraacetylglucosyl trichloroethanimidate, trimethylsilyl trifluoromethanesulfonate and a 4Å-type aluminosilicate molecular sieve, and stirring and reacting at 0°C for 10 h to obtain the compound of formula 15;

synthesis of a compound of formula 16 comprises: dissolving the compound of formula 15 in a mixed solution of dichloromethane and methanol, adding sodium methoxide, and stirring and reacting at 25°C for 4 h to obtain the compound of formula 16; and/or

synthesis of a compound of formula 17 comprises: dissolving the compound of formula 16 in anhydrous tetrahydrofuran, adding tetrabutylammonium fluoride, and stirring and reacting at 35°C for 3 h to obtain the compound of formula 17.

**INTERNATIONAL SEARCH REPORT**

International application No. **PCT/CN2022/134969**

**A. CLASSIFICATION OF SUBJECT MATTER**

A61K31/7048(2006.01)i;A61K31/34(2006.01)i;A61P31/16(2006.01)i;A61P31/14(2006.01)i;A61P29/00(2006.01)i;A61P11/00(2006.01)i;C07H1/00(2006.01)i

According to International Patent Classification (IPC) or to both national classification and IPC

**B. FIELDS SEARCHED**

Minimum documentation searched (classification system followed by classification symbols)

A61K,A61P,C07H

Documentation searched other than minimum documentation to the extent that such documents are included in the fields searched

Electronic data base consulted during the international search (name of data base and, where practicable, search terms used)

CNABS, DWPI, VEN, WPABS, WPABSC, CNTXT, ENTXT, ENTXTC, JPTXT, KRTXT, CJFD, WFB, WFCCP, WFCD, WFCJ, WFCOA, WFCSTA, WFFCP, WFFD, WFFJ, WFFOA, WFS, WFSTR, CNKI, STN, Web of Science, Elsevier Science Direct, PubMed, 百度学术, BAIDU SCHOLAR: 化合物结构式检索, compound structural formula search, 合成反应式检索, synthesis reaction formula search, 连翘苷, 连翘甙, 487-41-2, 连翘脂素, 连翘苷元, 连翘甙元, 487-39-8, 连翘新苷, 连翘新甙, 2231630-68-3, 流感, 副流感, 新型冠状病毒, 新冠, 肺出血, 肺炎, 木脂素, 双四氢呋喃, 双环氧木脂素, 合成, structure search, synthesis reaction search, phillyrin, phillygenin, Koreanaside A, influenza, flu, parainfluenza, PIV, HPIV, COVID-19, SARS-CoV-2, pulmonary hemorrhage, pneumorrhagia, pneumonia, lignan, furofuran, double-tetrahydrofuran, synthesis

**C. DOCUMENTS CONSIDERED TO BE RELEVANT**

| Category* | Citation of document, with indication, where appropriate, of the relevant passages | Relevant to claim No. |
|---|---|---|
| X | WO 2021179878 A1 (DALIAN FUSHENG NATURAL MEDICINE DEVELOPMENT CO., LTD.) 16 September 2021 (2021-09-16) claims 1-21, description, page 1, paragraph 2, page 3, paragraph 2, and figure 1 | 1-12 |
| X | CN 105362283 A (FU LI) 02 March 2016 (2016-03-02) claims 1-10, and description, paragraphs 164-167 and 204-221 | 1, 3-9, 11-12 |
| Y | CN 105362283 A (FU LI) 02 March 2016 (2016-03-02) claims 1-10, and description, paragraphs 164-167 and 204-221 | 6-9 |
| X | CN 105461767 A (FU LI) 06 April 2016 (2016-04-06) description, paragraphs 7-38 and 167 | 1, 3-7, 11-12 |
| Y | CN 105461767 A (FU LI) 06 April 2016 (2016-04-06) description, paragraphs 7-38 and 167 | 6-9, 13-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 February 2023** | **27 February 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/134969**

**C. DOCUMENTS CONSIDERED TO BE RELEVANT**

| Category* | Citation of document, with indication, where appropriate, of the relevant passages | Relevant to claim No. |
|---|---|---|
| X | CN 104650108 A (FU LI) 27 May 2015 (2015-05-27) claims 1-7, and description, paragraph 226 | 1, 3-5, 11-12 |
| Y | CN 104650108 A (FU LI) 27 May 2015 (2015-05-27) claims 1-7, and description, paragraph 226 | 6-9 |
| X | CN 106188176 A (FU LI) 07 December 2016 (2016-12-07) claims 1-10, and description, paragraph 258 | 1, 3-5, 11-12 |
| Y | CN 106188176 A (FU LI) 07 December 2016 (2016-12-07) claims 1-10, and description, paragraph 258 | 6-9 |
| X | CN 105461731 A (FU LI) 06 April 2016 (2016-04-06) claims 1-10, and description, paragraph 200 | 1, 3-5, 11-12 |
| Y | CN 105461731 A (FU LI) 06 April 2016 (2016-04-06) claims 1-10, and description, paragraph 200 | 6-9 |
| A | KR 102075482 B1 (UNIVERSITY-INDUSTRY COOPERATION GROUP OF KYUNG HEE UNIVERSITY) 10 February 2020 (2020-02-10) description, and abstract | 1-16 |
| A | KIM, T. W. et al. "Anti-Inflammatory Mechanisms of Koreanaside A, a Lignan Isolated from the Flower of Forsythia koreana, against LPS-Induced Macrophage Activation and DSS-Induced Colitis Mice: The Crucial Role of AP-1, NF-κB, and JAK/STAT Signaling." *CELLS,* Vol. 8, No. 10, 27 September 2019 (2019-09-27), ISSN: 2073-4409, abstract | 1-16 |
| Y | 陈宁等 (CHEN, Ning et al.). "芳基不同型双并四氢呋喃木脂素的新合成方法 (A New Synthetic Route to Furofuran Lignans with Two Different Aryl Groups—Synthesis of (±)-Methyl Piperitol)" *高等学校化学学报 (Chemical Journal of Chinese Universities),* Vol. 18, No. 03, 31 March 1997 (1997-03-31), ISSN: 0251-0790, page 416, and section 1 | 13-16 |

Form PCT/ISA/210 (second sheet) (July 2022)

**INTERNATIONAL SEARCH REPORT**
**Information on patent family members**

International application No.
**PCT/CN2022/134969**

| Patent document cited in search report | Publication date (day/month/year) | Patent family member(s) | Publication date (day/month/year) |
|---|---|---|---|
| WO 2021179878 A1 | 16 September 2021 | EP 4119146 A1 | 18 January 2023 |
| | | CA 3168457 A1 | 16 September 2021 |
| | | BR 112022015046 A2 | 20 September 2022 |
| CN 105362283 A | 02 March 2016 | RU 2655616 C1 | 29 May 2018 |
| | | WO 2016019684 A1 | 11 February 2016 |
| | | JP 2017523191 A | 17 August 2017 |
| | | JP 6343389 B2 | 13 June 2018 |
| | | US 2017232025 A1 | 17 August 2017 |
| | | US 10286002 B2 | 14 May 2019 |
| | | AU 2014402938 A1 | 23 March 2017 |
| | | AU 2014402938 B2 | 14 February 2019 |
| | | ES 2839090 T3 | 05 July 2021 |
| | | US 2019175635 A1 | 13 June 2019 |
| | | US 10881679 B2 | 05 January 2021 |
| | | EP 3178482 A1 | 14 June 2017 |
| | | EP 3178482 A4 | 11 April 2018 |
| | | EP 3178482 B1 | 30 September 2020 |
| | | KR 20170036098 A | 31 March 2017 |
| | | KR 101939726 B1 | 17 January 2019 |
| | | CA 2956988 A1 | 11 February 2016 |
| | | CA 2956988 C | 18 June 2019 |
| CN 105461767 A | 06 April 2016 | WO 2016019682 A1 | 11 February 2016 |
| | | US 2017233425 A1 | 17 August 2017 |
| | | US 10400004 B2 | 03 September 2019 |
| | | CA 2956980 A1 | 11 February 2016 |
| | | JP 2017523190 A | 17 August 2017 |
| | | JP 6305626 B2 | 04 April 2018 |
| | | AU 2014402936 A1 | 02 March 2017 |
| | | AU 2014402936 B2 | 31 May 2018 |
| | | RU 2017107144 A3 | 07 September 2018 |
| | | RU 2017107144 A | 25 September 2018 |
| | | KR 20170040324 A | 12 April 2017 |
| | | KR 102057549 B1 | 19 December 2019 |
| | | ES 2819302 T3 | 15 April 2021 |
| | | EP 3178834 A1 | 14 June 2017 |
| | | EP 3178834 A4 | 04 April 2018 |
| | | EP 3178834 B1 | 24 June 2020 |
| CN 104650108 A | 27 May 2015 | RU 2016124221 A | 25 December 2017 |
| | | RU 2642784 C2 | 26 January 2018 |
| | | ES 2749611 T3 | 23 March 2020 |
| | | EP 3072895 A1 | 28 September 2016 |
| | | EP 3072895 A4 | 31 May 2017 |
| | | EP 3072895 B1 | 07 August 2019 |
| | | JP 2016537359 A | 01 December 2016 |
| | | JP 6466444 B2 | 06 February 2019 |
| | | WO 2015070724 A1 | 21 May 2015 |
| | | CA 2930338 A1 | 21 May 2015 |
| | | CA 2930338 C | 21 August 2018 |
| | | AU 2014350785 A1 | 09 June 2016 |
| | | AU 2014350785 B2 | 14 September 2017 |

Form PCT/ISA/210 (patent family annex) (July 2022)

**INTERNATIONAL SEARCH REPORT**
**Information on patent family members**

International application No.
**PCT/CN2022/134969**

| Patent document cited in search report | | | Publication date (day/month/year) | Patent family member(s) | | | Publication date (day/month/year) |
|---|---|---|---|---|---|---|---|
| | | | | US | 2016355524 | A1 | 08 December 2016 |
| | | | | US | 9670224 | B2 | 06 June 2017 |
| | | | | KR | 20160085886 | A | 18 July 2016 |
| | | | | KR | 101850902 | B1 | 20 April 2018 |
| CN | 106188176 | A | 07 December 2016 | WO | 2016101733 | A1 | 30 June 2016 |
| CN | 105461731 | A | 06 April 2016 | CA | 2956982 | A1 | 11 February 2016 |
| | | | | CA | 2956982 | C | 11 June 2019 |
| | | | | AU | 2014402937 | A1 | 16 March 2017 |
| | | | | AU | 2014402937 | B2 | 28 June 2018 |
| | | | | JP | 2017523973 | A | 24 August 2017 |
| | | | | JP | 6310612 | B2 | 11 April 2018 |
| | | | | US | 2017233403 | A1 | 17 August 2017 |
| | | | | US | 9963461 | B2 | 08 May 2018 |
| | | | | RU | 2659072 | C1 | 28 June 2018 |
| | | | | ES | 2736604 | T3 | 03 January 2020 |
| | | | | EP | 3178822 | A1 | 14 June 2017 |
| | | | | EP | 3178822 | A4 | 24 January 2018 |
| | | | | EP | 3178822 | B1 | 13 March 2019 |
| | | | | WO | 2016019683 | A1 | 11 February 2016 |
| | | | | KR | 20170038914 | A | 07 April 2017 |
| | | | | KR | 101925133 | B1 | 05 December 2018 |
| KR | 102075482 | B1 | 10 February 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201310580801
- CN 201510320579
- CN 201410387045